Europäisches Patentamt

European Patent Office

Office européen des brevets

(11) Publication number: **0 225 286 B1**

## EUROPEAN PATENT SPECIFICATION

(45) Date of publication of patent specification: **22.07.92**

(51) Int. Cl.5: **C12N 15/58**, C12N 9/64, C12P 21/02, C12N 1/19, A61K 37/54

(21) Application number: **86810518.0**

(22) Date of filing: **13.11.86**

(54) Modified fibrinolytic agents.

(30) Priority: **18.11.85 GB 8528321**

(43) Date of publication of application:
**10.06.87 Bulletin 87/24**

(45) Publication of the grant of the patent:
**22.07.92 Bulletin 92/30**

(84) Designated Contracting States:
**AT BE CH DE ES FR GB GR IT LI LU NL SE**

(56) References cited:
**EP-A- 0 093 619**
**EP-A- 0 178 105**
**WO-A-84/01786**
**DE-A- 3 537 176**

**BIOTECHNOLOGY, vol. 2, no. 12, December 1984, pages 1051-1057, Macmillan Journals Ltd, London, GB; G.A. VEHAR et al.: "Characterization studies on human melanoma cell tissue plasminogen activator"**

**NATURE, vol. 301, no. 5897, January 1983, pages 214-221, Macmillan Journals Ltd, Lon-** don, GB; D. PENNICA et al.: "Cloning and expression of human tissue-type plasminogen activator cDNA in E. coli"

(73) Proprietor: **CIBA-GEIGY AG**
**Klybeckstrasse 141**
**CH-4002 Basel(CH)**

(72) Inventor: **Chaudhuri, Bhabatosh, Dr.**
**Maulbeerstrasse 15**
**CH-4058 Basle(CH)**
Inventor: **Meyhack, Bernd, Dr.**
**Höhenweg 9**
**CH-4312 Magden(CH)**
Inventor: **Heim, Jutta, Dr.**
**Rankackerweg 18**
**CH-4133 Pratteln(CH)**
Inventor: **van Oostrum, Jan, Dr.**
**Melchior Berristrasse 10**
**CH-4142 Münchenstein(CH)**

**Description**

Modified fibrinolytic agents

The invention relates to modified fibrinolytic agents and to means for the production of such fibrinolytic agents. More specifically, the invention relates to deglycosylated or partially deglycosylated tissue plasminogen activators, to hybrid vectors containing DNA sequences encoding such tissue plasminogen activators and to eukaryotic host cells transformed with said hybrid vectors. The invention concerns also the processes for the production of said hybrid vectors, said transformed host cells and said tissue plasminogen activators.

Background of the invention

Blood clots are the main cause of morbidity and of mortality of humans in the developed world. Blood clots are composed of fibrin which is formed from its soluble precursor fibrinogen by the action of the enzyme thrombin. An array of enzymes and other substances ensure that clots normally form only when and where they are required to prevent loss of blood.

Mammalian plasma contains an enzymatic system capable of dissolving the fibrin in blood clots. One component of the fibrinolytic system is a group of enzymes named plasminogen activators, which convert plasminogen (an inactive proenzyme form of plasmin) to the proteolytic enzyme plasmin. Plasmin then degrades the fibrin network of the clots to form soluble products. In cases where the thrombolytic potential of the body is insufficient to remove intravascular thrombi, for example in patients suffering from thromboembolisms or post-surgical complications, it may be indispensable to use exogenously administered thrombolytic agents.

Two types of plasminogen activators can be isolated from human body fluids or cells: urokinase, a serine protease occurring e.g. in human urine and kidney cells, and tissue plasminogen activator (hereinafter referred to as "t-PA") which is present in endothelial cells and a number of endocrine tissues. Tissue plasminogen activator differs from urokinase with respect to its chemical and immunological properties, in its greatly enhanced fibrinolytic action in the presence of fibrin, as well as in its high affinity for fibrin. t-PA exists in two molecular forms: the active two-chain form and a precursor one-chain form (often designated "pro t-PA"). The one-chain form can be converted to the two-chain form by incubation with catalytic amounts of plasmin preferably in the presence of fibrin.

The nucleotide sequence of cDNA coding for human t-PA has been determined and the amino acid sequence deduced therefrom [D. Pennica et al., Nature 301, 214 (1983)]. Four potential N-glycosylation sites have been identified, one of which is apparently not utilized for carbohydrate attachment [G. Pohl et al., Biochemistry 23, 3701 (1984)].

Sources of human t-PA include, for example, extracts of human tissues (which are not available for commercial exploitation) and various human tumor cells which have been shown to release t-PA to a varying extent. Thus, in European Patent Application No. 41,766 a t-PA is disclosed which has been isolated from a cultured human melanoma cell line. Recombinant DNA technology has also been made use of for the production of human t-PA. Double stranded t-PA cDNA derived from human tumour cells has been cloned and expressed in Chinese hamster ovary (CHO) cells and in E. coli (European Patent Application No. 93,619) or yeast (S. cerevisiae) cells (European Patent Applications Nos. 123,544 and 143,081). t-PA obtained from cultured human melanoma cells or from transformed CHO cells probably corresponds to the authentic t-PA glycoprotein. t-PA isolated from transformed yeast cells is likely to have carbohydrate chains specific for yeast. Differences in the structure of the carbohydrate chains of yeast and higher eucaryotes are well documented and relate to the outer chain addition of mannose residues and the modification of the core carbohydrates [cf. R. and S. Kornfeld, Ann.Rev.Biochem. 54, 631 (1985); J. C. Byrd et al., J. Biol. Chem. 257, 14657 (1982); R. B. Trimble et al., J. Biol. Chem. 258, 2562 (1983)]. The t-PA expressed in E. coli is not glycosylated but is fibrinolytically active although it has a rather low specific activity. Most of the protein accumulates in the cytoplasm in an inactive form, probably due to incorrect folding of the molecule. The correct folding depends on the formation of a number of specific disulfide bridges within the t-PA molecule which are essential for enzyme activity.

It has been shown that the carbohydrate residues of t-PA can be chemically degraded with oxidizing agents, such as sodium periodate (PCT Patent Applications No. 84/1786 and 84/1960). The resulting t-PA derivative is said to have a physiological clearance rate which is slower than that of untreated t-PA. The extent of chemical degradation of the carbohydrate radicals, the specific activity of the modified t-PA and the probably harmful chemical effect of the oxidising agent on sensitive amino acid radicals of the t-PA chain [it is expected that, for example, tryptophan residues are attacked by periodate; cf. A.S. Inglis et al., FEBS Lett. 104, 115 (1979)] are not disclosed.

S.P. Little et al. [Biochemistry 23, 6191 (1984)] describe the action of the glycosylation inhibitor tunicamycin on t-PA producing melanoma cells. They found that glycosylation is inhibited by 90 %. At the same time also protein synthesis is inhibited by 30 %, indicating that tunicamycin interferes directly or indirectly with vital processes in the cell. The resulting t-PA protein retains the fibrinolytic activity. However,the potential presence of traces of the deleterious glycosylation and protein biosynthesis inhibitor tunicamycin must necessarily limit the use of this t-PA as therapeutic agent. In the same paper the enzymatic deglycosylation of t-PA by endo-β-N-acetylglucosaminidase H (Endo H) is described. Endo H treatment leads to a mixture of t-PA species from which up to 85-90 % of the carbohydrate residues are removed. However, about 50 % of t-PA was completely resistant to Endo H treatment. The product is said to retain the fibrin-directed properties of authentic t-PA.

In view of the presumably beneficial properties of t-PA molecules lacking part or all of the carbohydrate residues there is a need for such compounds as well as for methods which allow the convenient synthesis of these proteins. Except for the product isolated from transformed E. coli cells (which, however, does not appear to be of great value for therapeutic purposes, supra) the deglycosylated t-PA species described in literature are chemically undefined molecules and, moreover, are present in complex mixtures containing an indefinite number of individual species. t-PA species in which one or all of the N-linked carbohydrate chains are totally removed are unknown so far.

Since all eukaryotes share the common mechanisms for the expression of genetic information, the expression of eukaryotic genes may proceed with greater efficiency in an eukaryotic host than in E. coli. Among the eukaryotic organisms suitable for exploitation yeast is the easiest to handle. Since yeast is a microorganism, yeast cells are easy to cultivate. The cell mass obtainable per volume of culture fluid is considerably higher for yeast than for E. coli and is free of endotoxins. In addition, the fermentational behaviour of yeast is well understood and conditions for large scale fermentations are already established. The secretory pathway of yeast resembles that of higher cells, including human cells. It is known that yeast cells can process their own proteins as well as foreign proteins by cleaving the corresponding signal sequences. In addition it seems that proteins which enter and pass through the secretory pathway of an eukaryotic host are likely to form a high degree of tertiary structure as compared to proteins synthesized in the cytoplasm. It is to be noted that prokaryotes such as E. coli do not seem to have large proteins with higher order structures.

Associated with the secretory pathway of higher organisms is the glycosylation system. It is expected that differences in glycosylation may influence sorting of the proteins. Thus, yeast carboxypeptidase Y which is simply core-glycosylated by 13 mannose residues is transferred to the vacuole whereas yeast invertase having outer carbohydrate chains consisting of about 100-150 mannose residues passes through the secretory vesicles and enters the periplasmic space [L. Lehle et al., J. Biol. Chem. 254, 12209 (1979); C. Hashimoto et al., Proc. Natl. Acad. Sci. USA 78, 2244 (1981)]. It is thus beyond question that altering the carbohydrate chains can have an influence on the secretory behaviour of t-PA.

Object of the invention

It is an object of the present invention to provide novel human t-PA proteins lacking one or more of the N-linked carbohydrate chains, however retaining valuable biological properties, such as fibrinolytic activity, a slower physiological clearance rate and prolonged in vivo stability than those of natural t-PA. Further objects of the invention are hybrid vectors containing DNA sequences encoding such t-PAs and a strong promoter system effectively controlling the expression of said DNA sequences.

Detailed description of the invention

The present invention provides novel human mutant t-PA proteins in which one or more of the naturally occurring N-glycosylation sites is (are) altered in such a manner that glycosylation cannot take place at these sites. This goal is achieved by providing DNA sequences coding for such t-PA proteins, hybrid vectors containing such DNA sequences and host cells transformed with such hybrid vectors and expressing such t-PA proteins.

1. Production of mature t-PA lacking one or more of the carbohydrate chains

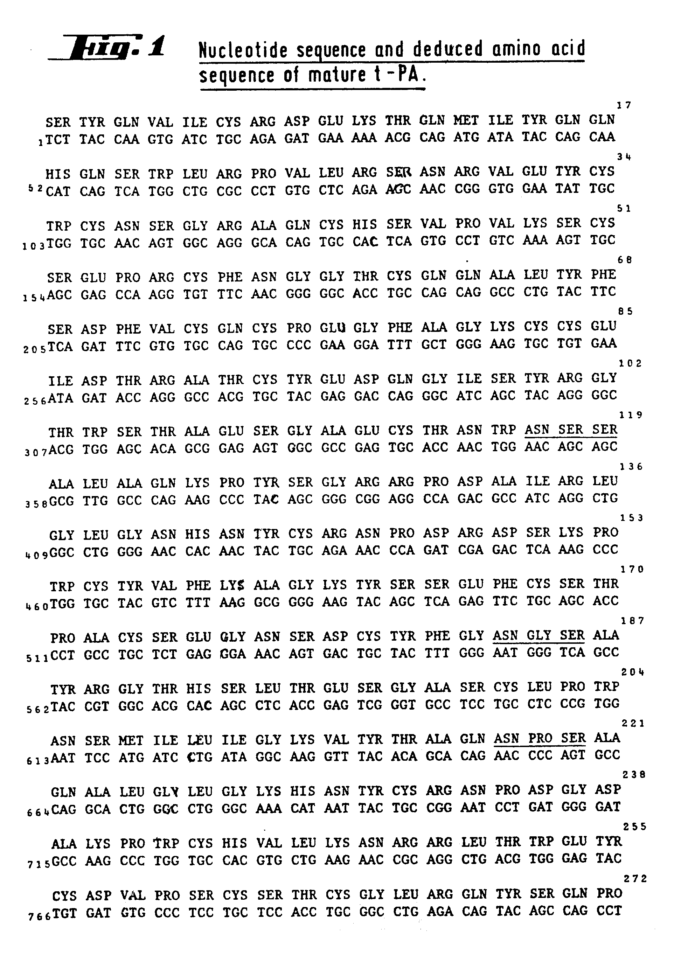

It is well established that a prerequisite for N-linked glycosylation in mammalian cells is the occurrence of the tripeptide sequence -Asn-L-Ser(or Thr)- wherein Asn is the acceptor and L can be any of the 20 genetically encoded amino acids except proline or aspartic acid which prevents glycosylation [c.f. D.K. Struck and W.J. Lennarz, in The Biochemistry of Glycoproteins and Proteoglycans, ed. W.J. Lennarz, Plenum Press 1980, p. 35; R.D. Marshall, Biochem. Soc. Symp. 40, 17 (1974)]. There are four potential sites for N-glycosidic linkage in the t-PA molecule (the numbers refer to the position of Asn in the amino acid sequence of t-PA, cf. Fig. 1 of the accompanying drawings): -$Asn^{117}$-Ser-Ser-, $Asn^{184}$-Gly-Ser-, $Asn^{218}$-Pro-Ser and $Asn^{448}$-Arg-Thr-. Because of the presence of Pro in the sequence $Asn^{218}$-Pro-Ser, $Asn^{218}$ is not utilized for carbohydrate attachment in mammalian cells (G. Pohl et al., supra).

In order to prevent glycosylation at individual (one or more of the) N-glycosylation sites the tripeptide sequences recognised as signals for N-glycosylation have to be altered. Replacement of the Asn and/or Ser (or Thr) residues in the above tripeptide sequences by any other amino acid would abolish formation of glycosidic linkages at these sites. For convenience, modification of the t-PA molecule, i.e. alteration of the N-glycosylation sites, is not done at the protein level. Instead, it is advantageous to modify the gene coding for t-PA in such a way that upon expression of said modified gene by a host a mutant t-PA is produced in which one or more of the naturally occurring N-glycosylation sites are altered in such a way that glycosylation cannot take place at these sites.

In particular, the invention relates to human mutant t-PA proteins having the amino acid sequence

$A_{1-116}$-Asn-Ser-$Y_1$-$A_{120-183}$-Asn-Gly-$Y_2$-$A_{187-217}$-Asn-Pro-$Y_3$-$A_{221-447}$-Asn-Arg-$Y_4$-$A_{451-527}$     (I')

in which $A_{1-116}$ represents the N-terminal amino acid sequence consisting of amino acids 1 to 116 of mature t-PA, $A_{120-183}$ represents the amino acid sequence consisting of amino acids 120 to 183 of mature t-PA, $A_{187-217}$ represents the amino acid sequence consisting of amino acids 187 to 217 of mature t-PA, $A_{221-447}$ represents the amino acid sequence consisting of amino acids 221 to 447 of mature TPA, $A_{451-527}$ represents the C-terminal amino acid sequence consisting of amino acids 451 to 527 of mature t-PA, and

a) $Y_1$ and $Y_3$ are Ser, $Y_2$ is a genetically encoded amino acid other than Ser and Thr, and $Y_4$ is Thr; or

b) $Y_1$, $Y_2$ and $Y_3$ are each Ser and $Y_4$ is a genetically encoded amino acid other than Ser and Thr; or

c) $Y_1$, $Y_2$ and $Y_4$ are each independently a genetically encoded amino acid other than Ser and Thr, and $Y_3$ is Ser, in the one-chain or in the two-chain form.

The term "mature t-PA" refers to a t-PA which is devoid of any pre-or pro-sequences starting with Ser in position 1. Reference is made to Figure 1 of the accompanying drawings showing the amino acid sequence of mature t-PA.

Genetically encoded amino acids are the following L-amino acids: Ala, Ser, Thr, Val, Leu, Ile, Asp, Asn, Glu, Gln, Cys, Met, Arg, Lys, His, Tyr, Phe, Trp and Pro, furthermore the amino acid Gly.

For replacement those amino acids are preferred which have a similar size and polarity as the amino acids to be replaced. The number of possible substituents of the Thr or Ser residues is limited by the given nucleotide sequence and the possible codons (cf. paragraph 2). For replacement of Ser $Y_1$, $Y_2$ and/or $Y_3$ and for the replacement of Thr $Y_4$ amino acids Ala and Asn are preferred.

In particular, the invention relates to a t-PA protein selected from the group consisting of [$Ala^{186}$]-t-PA, [$Ala^{450}$]-t-PA, [$Asn^{119}$, $Ala^{186}$, $Asn^{450}$]-t-PA and [$Asn^{119}$, $Ala^{186}$, $Ala^{450}$]-t-PA.

Compounds of the formula I in which at least one of the original t-PA N-glycosylation sites is retained are N-glycosylated. Compounds of the formula I in which no one of the original t-PA glycosylation sites is retained are devoid of any N-linked glycosylation.

The one-chain form of the t-PA mutants according to the invention corresponds to formula I depicted above. The two-chain form is generated by cleavage of the $Arg^{275}$ $Ile^{276}$ peptide bond (cf. Figure 1). The two chains are held together by means of a disulphide bridge. Whenever used in the present invention and unless expressly defined otherwise, the term "t-PA" refers to the one-chain form which is the preferred form of the t-PAs according to the invention.

The compounds of the formula I are preferably made by biotechnological means.

The method for the production of mutant t-PA proteins according to the invention comprises culturing under appropriate nutrient conditions transformed eukaryotic host cells containing a DNA sequence coding for any of said mutant t-PA proteins and isolating said mutant t-PA protein and, if desired, separating a mixture of the one-chain and two-chain forms of the mutant t-PA obtained into the individual components, and for the production of the two-chain form which is free of the one-chain form, converting the one-chain form of the t-PA mutant produced into the two-chain form.

The primary product of t-PA gene expression according to the present invention is the one-chain form of t-PA. If, however, proteases are present either in the host cell or in the culture fluid, the primarily expressed one-chain t-PA may at least partially be converted into two-chain t-PA. The actually isolated product may therefore consist of mutant one-chain t-PA, mutant two-chain t-PA or mixtures thereof.

Suitable eukaryotic host cells are cells of higher organisms, especially mammalians, in particular established human or animal cell lines, such as the human Bowes cell line or HeLa cells, or the Chinese hamster ovary (CHO) cell line, and yeast, such as Saccharomyces cerevisiae. The preferred host cells according to the present invention are cells of Saccharomyces cerevisiae.

The eukaryotic host cells especially yeast cells, containing the above DNA sequence are cultured using methods known in the art.

Thus, transformed yeast strains according to the invention are cultured in a liquid medium containing assimilable sources of carbon, nitrogen and inorganic salts.

Various carbon sources are usable. Examples of preferred carbon sources are assimilable carbohydrates, such as glucose, maltose, mannitol or lactose, or an acetate such as sodium acetate, which can be used either alone or in suitable mixtures. Suitable nitrogen sources include, for example, amino acids, such as casamino acids, peptides and proteins and their degradation products, such as tryptone, peptone or meat extracts, furthermore yeast extract, malt extract, corn steep liquor, as well as ammonium salts, such as ammonium chloride, sulphate or nitrate, which can be used either alone or in suitable mixtures. Inorganic salts which may be used include, for example, sulphates, chlorides, phosphates and carbonates of sodium, potassium, magnesium and calcium. Additionally, the nutrient medium may also contain growth promoting substances. Substances which promote growth include, for example, trace elements, such as iron, zinc, manganese and the like, or individual amino acids.

To a varying extent, yeast cells transformed with autonomously replicating plasmids, for example, plasmids containing yeast 2μ plasmid DNA (infra) tend to lose the introduced hybrid plasmid. For this reason, such yeast cells have to be grown under selective conditions, i.e. conditions which require the expression of a plasmid-encoded gene for growth. Most selective markers currently in use and present in the hybrid vectors according to the invention (infra) are genes coding for enzymes of amino acid or purine biosynthesis. This makes it necessary to use synthetic minimal media deficient in the corresponding amino acid or purine base. However, some genes conferring resistance to an appropriate biocide may be used as well [e.g. genes conferring resistance to cycloheximide, to the amino-glycoside G 418, to a heavy metal, or the like]. Yeast cells transformed with vectors containing antibiotic resistance genes are grown in complex media containing the corresponding antibiotic whereby faster growth rates and higher cell densities are reached.

Yeast cells transformed with DNA integrating into the chromosomes do not require selective growth conditions. These transformed cells are sufficiently stable to allow growth without selective pressure. For the above reason, these cells are advantageously grown in complex media.

Yeast cells containing hybrid plasmids with a constitutive promoter (e.g. ADHI, GAPDH) express the t-PA gene attached to said promoter without induction. However, if the t-PA gene is under the control of a regulated promoter (e.g. PGK or PHO5) the composition of the growth medium has to be adapted in order to obtain maximum levels of mRNA transcripts, i.e. when using the PHO5 promoter the growth medium must contain a low concentration of inorganic phosphate for derepression of this promoter.

The cultivation is carried out by employing conventional techniques. The culturing conditions, such as temperature, pH of the medium and fermentation time are selected in such a way that maximal levels of t-PA are produced. A chosen yeast strain is preferably grown under aerobic conditions in submerged culture with shaking or stirring at a temperature of about 25° to 35°C, preferably at about 30°C, at a pH value of from 4 to 8, for example at approximately pH 7, and for about 4 to 20 hours, preferably until maximum yields of proteins are reached.

The produced t-PA protein can accumulate within the yeast cells or can be secreted into the periplasmic space or into the culture medium. In case the t-PA protein has accumulated within the cells, the first step for the recovery of the t-PA protein consists in liberating the protein from the cell interior. In most procedures the cell wall is first removed by enzymatic digestion with glucosidases (cf. section 4). Subsequently, the resulting spheroplasts are treated with detergents, such as Triton® X-100. Alternatively, mechanical forces, such as shearing forces (for example X-press, French-press) or shaking with glass beads, are suitable for breaking cells. The resulting mixture is enriched for mutant t-PA by conventional means, such as removal of most of the non-proteinaceous material by treatment with polyethyleneimine, precipitation of the proteins using ammonium sulphate or trichloroacetic acid, gel electrophoresis, dialysis, chromatography, for example, ion exchange chromatography, size-exclusion chromatography, HPLC or reverse phase HPLC, molecular sizing on a suitable Sephadex® column, or the like. The final purification of the pre-purified product is achieved, for example, by means of affinity chromatography, for example antibody affinity chromatography, especially monoclonal antibody affinity chromatography using monoclonal anti-t-PA antibodies fixed on an insoluble matrix by methods known in the art, and the like. Further preferred methods for isolating the mutant t-PA from culture

fluids consist in selectively adsorbing it on a support of specific affinity of soluble fibrin fragments which

EP 0 225 286 B1

are covalently fixed on an insoluble matrix, e.g. dextran or, in particular, on DE-3 Sepharose®. DE-3 is a trypsin inhibitor which is contained in the seeds of the legume Erythrina latissima (European Patent Application No. 112,122). It has been found that DE-3 is also able to inhibit t-PA activity. Thus, the purified DE-3 inhibitor may be coupled to an insoluble matrix, e.g. BrCN activated Sepharose®, using standard procedures. t-PA proteins will be adsorbed to the inhibitor matrix and can be eluted by a buffer which contains a chaotropic agent.

In a preferred embodiment of the present invention, the culture medium, optionally after having passed through one or more of the above-mentioned purification steps, is passed at room temperature through a column or is treated in a batch procedure with BrCN activated Sepharose® to which the DE-3 inhibitor has been coupled. After washing the Sepharose® support, the adsorbed tissue plasminogen activator is eluted by treatment with a buffer solution, for example phosphate buffered saline, having a pH of approximately 5.5-6.0 and containing a chaotropic agent, such as $NH_4SCN$, from about 1.0 to about 2.0 molar, preferably 1.2 molar. Alternatively, benzamidine or arginine may be chosen as releasing agent. Advantageously, a detergent, especially a nonionic detergent, such as Triton X-100® or Tween 80®, is added to all buffer solutions used in the purification steps, in order to prevent the adsorption of tissue plasminogen activator to the vessel surfaces and to improve stability. The detergent may be added to a final concentration of 0.01-0.1 %.

In the case where the tissue plasminogen activator is secreted by the yeast cell into the periplasmic space, a simplified protocol can be used: The protein is recovered without cell lysis by enzymatic removal of the cell wall or by treatment with chemical agents, e.g. thiol reagents or EDTA, which give rise to cell wall damages permitting the produced mutant t-PA to be released. In the case where the mutant t-PA is secreted into the culture broth, it can be recovered directly therefrom.

Cultivation of yeast cells harboring a chromosomally integrated t-PA gene and isolation and purification of the expressed t-PA is effected in the same way as described above.

When the preferred yeast strains according to the invention, viz. protease-deficient yeast strains, are used the isolated mutant t-PA is mostly in the one-chain form. When yeast strains are used which show proteolytic activity a product mixture consisting of the one-chain and the two-chain forms of the t-PA mutant is obtained in varying ratios.

For the preparation of the two-chain form of the t-PA mutant which is substantially free of the one-chain form, the one-chain form is enzymatically converted into the two-chain form by cleavage of the $Arg^{275}$-$Ile^{276}$ peptide bond, for example by the action of plasmin or an enzyme having an equivalent effect on the one-chain form of t-PA.

For the convenient preparation of the one-chain form of the mutant t-PA which is substantially free of the two-chain form, a protease inhibitor, such as aprotinin (Trasylol®) or basic pancreatic trypsin inhibitor, is advantageously included during the purification procedure in order to inhibit traces of proteases which may be present and which may cause (partial) conversion of the one-chain form into the two-chain form. The final purification is then achieved by chromatography on a column containing a selective affinity reagent, such as DE-3.

The transformed host cells according to the invention can be prepared by recombinant DNA techniques comprising the steps of

- preparing a structural gene coding for the mutant t-PA protein according to the invention,
- incorporating the obtained structural gene into an appropriate vector,
- transforming suitable host cells with the created hybrid vector
- and selecting transformed host cells from untransformed host cells.

## 2. DNA sequences coding for mutant t-PA proteins

The invention concerns also a DNA having a sequence coding for a human mutant t-PA protein according to the invention.

Specifically, the invention relates to a DNA having a sequence of the formula

$N_{1-348}$-AAC-AGC-$W_1$-$N_{348-549}$-AAT-GGG-$W_2$-$N_{559-651}$-AAC-CCC-$W_3$-$N_{661-1341}$-AAC-AGA-$W_4$-$N_{1351-1581}$ (II')

in which $N_{1-348}$ represents a deoxyribonucleotide sequence consisting of deoxyribonucleotides 1 to 348 of the gene for mature t-PA,

$N_{358-549}$ represents a deoxyribonucleotide sequence consisting of deoxyribonucleotides 358 to 549 of the gene for mature t-PA,

$N_{559-651}$ represents a deoxyribonucleotide sequence consisting of deoxyribonucleotides 559 to 651 of the gene for mature t-PA,

$N_{661-1341}$ represents a deoxyribonucleotide sequence consisting of deoxyribonucleotides 661 to 1341 of the gene for mature t-PA, and

$N_{1351-1581}$ represents a deoxyribonucleotide sequence consisting of deoxyribonucleotides 1351 to 1581 of the gene for mature t-PA, $W_1$, $W_2$, $W_3$ and $W_4$ each represent codons encoding amino acids $Y_1$, $Y_2$, $Y_3$ and $Y_4$, respectively, as defined above.

The gene coding for human mature t-PA is known and may be derived from any human cell which is able to produce t-PA. Such cells are of cancerous or non-cancerous origin and are preferably derived from an established cell line. In a preferred embodiment of the present invention the gene for t-PA is derived from HeLa cells. Reference is made to Figure 1 of the accompanying drawings in which the DNA sequence of mature t-PA occurring in HeLa cells starting with TCT coding for Ser as the first amino acid and numbering of the deoxyribonuleotides is depicted.

In wild-type mature t-PA $W_1$ is AGC, $W_2$ is TCA, $W_3$ is AGT and $W_4$ is ACA. In the compounds of the formula II' at least one of these original codons is altered in such a manner that the resulting codon encodes an amino acid $Y_1$, $Y_2$, $Y_3$ or $Y_4$ as defined above. The codons $W_1$, $W_2$, $W_3$ and/or $W_4$ can be replaced by any other codon which is different from a nonsense codon taking the above proviso into account. Those altered codons are preferred which code for the preferred amino acids detailed above and which differ from the original codons by one or, at the most, two deoxyribonucleotides.

In the compounds of the formula II' the codons $W_1$, $W_2$, $W_3$ and/or $W_4$ are preferably modified as follows: AGC ($W_1$, codes for Ser) is changed to ATC (Ile) or AAC (Asn), TCA ($W_2$, codes for Ser) is changed to GCA (Ala) or TTA (Leu), AGT ($W_3$, codes for Ser) is changed to AAT (Asn) or ATT (Ile), and ACA ($W_4$, codes for Thr) is changed to ATA (Ile), GCA (Ala), AAC or AAT (both Asn).

The DNA sequences of the formula II' are preferably those which code for the mutant t-PA proteins described hereinbefore as being especially preferred.

The DNAs having the sequences of the formula II' can be manufactured by methods known in the art. The methods for the manufacture of these DNA include chemically synthesizing the DNA, excising a portion of the DNA comprising the codon for the undesired amino acid residue from the parental mature t-PA gene and replacing it with a DNA segment wherein said codon has been substituted with a deoxyribonucleotide triplet coding for the desired amino acid residue, or accomplishing the deoxyribonucleotide substitution by means of site-directed mutagenesis.

The chemical synthesis of DNA is well-known in the art and makes use of conventional techniques. Appropriate techniques have been compiled by S.A. Narang [Tetrahedron 39, 3 (1983)]. In particular, the methods described in New Zealand Patent Specification No. 210267 may be used and are herein incorporated by reference.

Excision of a portion of the mature t-PA DNA may be effected by using restriction enzymes. A prerequisite of this method is the availability of appropriate restriction sites in the vicinity of the codon to be altered. A small restriction fragment containing the codon for an undesired amino acid is removed by endonuclease cleavage. A corresponding double stranded DNA sequence is prepared, for example by means of chemical synthesis, in which triplets coding for the desired amino acid are used. The DNA fragment is ligated in the proper orientation to the remaining large fragment to yield a double stranded DNA sequence coding for a mutant t-PA. For convenience and in order to facilitate handling of the t-PA gene the latter is advantageously contained in a greater DNA segment provided with appropriate linkers which allow insertion and cloning of the segment in a cloning vector.

In a preferred embodiment of the present invention the preparation of DNAs having the DNA sequences of the formula II' is effected by site-directed mutagenesis. This method is an in vitro mutagenesis procedure by which a defined site within a region of cloned DNA can be altered [cf. the review articles of M.J. Zoller and M. Smith, Methods Enzymol. 100, 468 (1983); D. Botstein and D. Shortle, Science 229, 1193 (1985)].

The method of mutating the wild-type t-PA gene is characterized in that the single-stranded wild-type t-PA gene or a single-stranded DNA comprising the wild-type t-PA gene is hybridized to an oligodeoxyribonucleotide primer which is complementary to the region of the t-PA gene to be mutated except for mismatch(es) that direct(s) the mutation, the hybridized oligodeoxyribonucleotide is used as a primer to initiate the synthesis of the complementary DNA strand, the resulting (partially) double-stranded DNA is transformed into a recipient microorganism strain, the microorganism strain is cultivated and transformants containing DNA with the modified t-PA gene are selected.

The wild-type t-PA gene is preferably contained in a plasmid, for example a plasmid described in New Zealand Patent Specification No. 210266. For mutagenesis the t-PA gene is advantageously isolated from the plasmid, for example by restriction endonuclease digestion resulting in a double-stranded DNA comprising the t-PA gene and optionally other functions linked thereto, for example promoter, signal peptide DNA etc, and having sticky ends, and is ligated to a suitable restriction fragment of the replicative form (RF) of a single-stranded DNA phage, such as a derivative of phage M13, e.g. M13mp8 or M13mp9. Competent microorganisms, e.g. Ca-treated cells of an E. coli strain, are transfected with the hybrid phage vector and single-stranded phage DNA containing the inserted t-PA gene is isolated from the supernatant of the cultured cells. To this template the mismatch (mutagenic) oligodeoxyribonucleotide primer is hybridized.

Some of the methods described in the literature depend on the enzymatic extension of the mutagenic primer along the circular template followed by ligation to produce a covalently closed double-stranded circular molecule having the mutation(s) in the newly synthesized strand. Due to the generally low efficiency of this process a modified procedure has been developed [K. Norris et al., Nucl. Acids Res. 11, 5103 (1983)] in which a second primer is used. In the case of an M13 phage vector a universal M13 sequencing primer is preferably used.

Accordingly, the phosphorylated mutagenic primer and the M13 sequencing primer are simultaneously annealed to the circular single-stranded t-PA template DNA. Annealing occurs rapidly at 55°C for 5 minutes and then at room temperature for 5 minutes. Advantageously, the primer-template molar ratio is about 20:1. Extension of both primers is carried out by E. coli DNA polymerase (Klenow fragment). The newly synthesized DNA strand from the sequencing primer is ligated to the 5' phosphorylated end of the mutagenic primer by T4 DNA ligase. This procedure forms partially double stranded molecules bearing the desired mismatch(es).

The mutagenic primer and the M13 sequencing primer can be chemically synthesized (supra). In order to sufficiently prime DNA synthesis at room temperature and above it is preferable to utilise oligodeoxyribonucleotides ranging in length from about 14 to 22 nucleotides. In addition, such oligodeoxyribonucleotides are more likely to recognize the unique target sites. Especially, a mutagenic oligodeoxyribonucleotide having one mismatch ranges in length from 14 to 18 nucleotides whilst mutagenic oligodeoxynucleotides having two mismatches range in length from about 17 to 30 nucleotides. The mutagenic primers are designed in such a manner that the mismatch(es) is (are) located near the middle of the molecule. This yields the greatest binding differential between a perfectly matched duplex and a mismatched duplex (infra).

In a preferred embodiment of the present invention, the mutagenic oligodeoxyribonucleotides contain triplets with one or two mismatches coding for amino acid residues which prevent glycosylation and are described hereinbefore as being especially preferred.

The partially double stranded DNA bearing the desired mismatch(es) can directly be used to transfect a recipient microorganism, especially a strain of E. coli. Transfection with the DNA results in a mixture of phages containing the wild-type t-PA gene and the mutated t-PA gene, respectively. The resulting plaques are screened for phage DNA containing the mutated t-PA gene.

A preferred method for selecting phage DNA containing the mutated t-PA gene from the resulting mixture of phages having wild-type or mutated t-PA incorporated is the so-called dot blot hybridization (cf. M.J. Zoller and M. Smith, supra). The method is based on the difference in thermal stability of a perfectly matched DNA duplex and a DNA duplex with one or more mismatches. The single-stranded phage DNA is immobilized on a solid support such as filter paper or nitrocellulose sheets by baking at elevated temperature, e.g. at 80°C, and hybridizing to the $^{32}$P labeled mutagenic oligodeoxyribonucleotide primer (supra). At low temperature both mutant and wild-type t-PA DNA interact by forming duplexes. Subsequently washes are carried out at increasingly higher temperatures (e.g. at intervals of 10°C) whereby the primer is selectively dissociating from the wild-type t-PA DNA until only perfectly matched mutant molecules remain hybridized. The mutant molecules are identified by autoradiography. The necessary final temperature can be calculated theoretically. Preferably, a temperature exceeding the melting temperature $T_o$ (point of 50% dissociation of the primer) by 4 to 8°C is chosen. The $T_o$ temperature is calculated by adding an increment of 2° for each AT base pair and an increment of 4° for each GC base pair of the perfectly base-paired primer-mutated t-PA gene duplex between the oligodeoxyribonucleotide primer and the mutated t-PA gene.

Pure mutant phages are obtained by retransfection of a recipient microorganism strain, such as an E. coli strain, with the identified primary mutant phages and again screening of the plaques for phage DNA containing the mutated t-PA gene by dot blot hybridization using the above mutagenic oligodeoxyribonucleotide primer ($^{32}$P labeled).

The mutated t-PA gene is excised from the replicative form of the phage by means of suitable restriction endonucleases and is used for cloning in a hybrid vector.

3. Hybrid vectors containing DNA sequences coding for mutant t-PA proteins

The invention relates also to hybrid vectors comprising a promoter and a DNA sequence coding for a human mutant t-PA protein according to the invention, which DNA sequence is under transcriptional control of said promoter.

The hybrid vectors according to the invention are selected from the group consisting of a hybrid plasmid and a linear DNA vector and are further selected depending on the host organism envisaged for transformation.

Promoters of mammalian cells are for example viral promoters, such as HTLV, SV40, vaccinia promoter and the like.

Promoters for yeast which are the most preferred host cells according to the present invention are derived from the genomic DNA of yeast, especially of Saccharomyces cerevisiae. Preferably, the promoter of a highly expressed yeast gene is used for the expression of t-PA mutants. Thus, the promoter of the TRP1 gene, the ADHI or ADHII gene, acid phosphatase (PHO5) gene, isocytochrome c gene, or a promoter of the genes coding for glycolytic enzymes, such as the promoter of the enolase, glyceraldehyde-3-phosphate dehydrogenase (GAPDH), 3-phosphoglycerate kinase (PGK), hexokinase, pyruvate decarboxylase, phosphofructokinase, glucose-6-phosphate isomerase, 3-phosphoglycerate mutase, pyruvate kinase, triosephosphate isomerase, phosphoglucose isomerase, invertase and glucokinase genes, or a promoter of the yeast mating pheromone genes coding for the a-or α-factor, can be used. Preferred vectors of the present invention contain promoters with transcriptional control. Promoters of this type, e.g. the promoter of the PHO5 gene, can be turned on or off by variation of the growth conditions. For example, the PHO5 promoter can be repressed or derepressed at will, solely by increasing or decreasing the concentration of inorganic phosphate in the medium. Thus, the promoter can be repressed during the exponential growth phase of the yeast and may be turned on (derepressed) only during early stationary phase at maximal cell density allowing expression of the gene controlled by the PHO5 promoter. This property combined with a high level of transcription makes the PHO5 promoter the preferred one in the present invention.

In the hybrid vectors of the present invention, the promoter is operably linked to the mutant t-PA coding region so as to ensure effective expression of the mutant t-PA. In one preferred embodiment of the present invention, the promoter is directly linked to the coding region of the mature t-PA mutant with a translation start signal (ATG) inserted at the junction. In another preferred embodiment of the present invention, especially if yeast is used as the host organism, a signal sequence is included in the construction. Suitable signal sequences are those naturally linked to the promoter used, especially the PHO5 or invertase signal sequence, or the t-PA signal sequence. Other signal sequences, such as those of the yeast invertase or α-factor genes, may also be used. Alternatively, fused signal sequences may be constructed by ligating part of the signal sequence of the gene naturally linked to the promoter used, with part of the t-PA signal sequence. Those combinations are favoured which allow a precise cleavage between the signal sequence and the mature t-PA amino acid sequence. Additional sequences, such as pro-or spacer-sequences which may or may not carry specific processing signals can also be included in the constructions to facilitate accurate processing of precursor molecules. Alternatively fused proteins can be generated containing internal processing signals which allow proper maturation in vivo or in vitro. Preferably, the processing signals contain a Lys-Arg residue, which is recognized by a yeast endopeptidase located in the Golgi membranes. Upon expression of the mutant t-PA gene, the gene product enters the secretory pathway and is transported to the periplasmic space. If further excretion through the cell wall into the culture medium can be achieved, a considerable increase in yields should be possible. Also the recovery process can be simplified with no cells to be disrupted.

Preferably, the hybrid vectors according to the present invention comprise also the 3'-flanking sequence of a gene which contains the proper signals for transcription termination and polyadenylation. Suitable 3'-flanking sequences are for example those of the gene naturally linked to the promoter used, in the case of yeast especially flanking sequences of the PKO5 gene.

The invention relates especially to a linear DNA vector consisting of a DNA sequence coding for a human mutant t-PA protein according to the invention, optionally including a signal sequence, flanked by sequences naturally occurring in a chromosomal gene, such as the promoter region of a host chromosomal gene at the 5'-end and (part of) the 3'-flanking region of said gene at the 3'-end. Especially, the invention relates to a linear DNA comprising the yeast PHO5 promoter, the mutant t-PA coding region and the PHO5 3'-flanking region.

By virtue of the homologous 3' and 5' flanking sequences the whole linear DNA vector including the mutant t-PA gene is stably integrated into the respective host chromosome, i.e. in case of the yeast PHO5 promoter and 3' flanking sequences of the yeast PHO5 gene at the PHO5 locus in yeast chromosome II.

The invention relates also especially to circular hybrid plasmids which apart from the promoter, a DNA sequence coding for a human mutant t-PA protein according to the invention, optionally including a signal sequence, and 3' flanking sequences contain additional DNA sequence(s) which are inessential or less important for the function of the promoter, i.e. for the expression of the modified t-PA coding region, but which perform important functions, for example, in the propagation of the cells transformed with said hybrid plasmids. The additional DNA sequence(s) may be derived from prokaryotic and/or eukaryotic cells and may include chromosomal and/or extra-chromosomal DNA sequences. For example, the additional DNA sequences may stem from (or consist of) plasmid DNA, such as bacterial or eukaryotic plasmid DNA, viral DNA and/or chromosomal DNA, such as bacterial, yeast or higher eukaryotic chromosomal DNA. Preferred hybrid plasmids contain additional DNA sequences derived from bacterial plasmids, especially Escherichia coli plasmid pBR322 or related plasmids, bacteriophage λ, yeast 2μ plasmid, and/or yeast chromosomal DNA.

In the preferred hybrid plasmids according to the invention, the additional DNA sequences carry a yeast replication origin and a selective genetic marker for yeast. Hybrid plasmids containing a yeast replication origin, e.g. an autonomously replicating segment(ars), are extrachromosomally maintained within the yeast cell after transformation and are autonomously replicated upon mitosis. Hybrid plasmids containing sequences homologous to yeast 2μ plasmid DNA can be used as well. These hybrid plasmids will get integrated by recombination into 2μ plasmids already present within the cell or will replicate autonomously. 2μ sequences are especially suitable for high-frequency transformation plasmids and give rise to high copy numbers.

In addition, the preferred hybrid plasmids according to the invention may include a DNA sequence as part of a gene present in the host yeast chromosome (e.g. the PHO5 gene or its promoter linked to the mutant t-PA coding region). By virtue of the homologous sequence, which should amount to a stretch of about 20 to 100 deoxynucleotides, the whole plasmid or linear fragments thereof can be stably incorporated into the host chromosome by recombination. Thus, during propagation the progeny cells will retain the introduced genetic material even without selective pressure.

As to the selective gene marker for yeast, any marker gene can be used which facilitates the selection for transformants due to the phenotypic expression of the marker. Suitable markers for yeast are particularly those expressing antibiotic resistance or, in the case of auxotrophic yeast mutants, genes which complement host lesions. Corresponding genes confer, for example, resistance to the antibiotic cycloheximide or provide for prototrophy in an auxotrophic yeast mutant, for example the URA3, LEU2, HIS3 or TRP1 gene. It is also possible to employ as markers structural genes which are associated with an autonomously replicating segment providing that the host to be transformed is auxotrophic for the product expressed by the marker.

Advantageously, the additional DNA sequences which are present in the hybrid plasmids according to the invention also include a replication origin and a selective genetic marker for a bacterial host, especially Escherichia coli. There are useful features which are associated with the presence of an E. coli replication origin and an E. coli marker in a yeast hybrid plasmid. Firstly, large amounts of hybrid plasmid DNA can be obtained by growth and amplification in E. coli and, secondly, the construction of hybrid plasmids is conveniently done in E. coli making use of the whole repertoire of cloning technology based on E. coli. E. coli plasmids, such as pBR322 and the like, contain both E. coli replication origin and E. coli genetic markers conferring resistance to antibiotics, for example tetracycline and ampicillin, and are advantagously employed as part of the yeast hybrid vectors.

The additional DNA sequences which contain, for example, replication origin and genetic markers for yeast and a bacterial host (see above) are hereinafter referred to as "vector DNA" which together with the yeast promoter and the mutant t-PA coding region is forming a hybrid plasmid according to the invention.

In a preferred embodiment, the present invention relates to hybrid plasmids capable of autonomous replication in a yeast host strain and having selectable markers and to linear DNAs which integrate into the host chromosome comprising a yeast promoter and a DNA sequence coding for a human mutant t-PA protein according to the invention, said DNA sequence being positioned together with transcription start and termination signals as well as encoded translation start and stop signals in said hybrid vector under control of said promoter such that in a transformed yeast strain it is expressed to produce said mutant tissue plasminogen activator.

The preferred hybrid vectors of the present invention are prepared by methods known in the art, for example by linking a yeast promoter, a mutant t-PA coding region, the 3' flanking sequence of a yeast gene and optionally vector DNA.

For the preparation of hybrid plasmids, conveniently mapped circular vector DNA, for example bacterial plasmid DNA or the like (see above), having at least one restriction site, preferably two or more restriction sites, can be employed. Advantageously, the vector DNA already contains replication origins and gene markers for yeast and/or a bacterial host. The vector DNA is cleaved using an appropriate restriction endonuclease. The restricted DNA is ligated to the DNA fragment containing the yeast promoter and to the DNA segment coding for the mutant t-PA. Prior to or after linking of the promoter and the mutant t-PA coding region (or simultaneously as well), it is also possible to introduce replication origins and/or markers for yeast or a bacterial host. At all events, the restriction and ligation conditions are to be chosen in such a manner that there is no interference with the essential functions of the vector DNA and of the promoter. The hybrid vector may be built up sequentially or by ligating two DNA segments comprising all sequences of interest.

Various techniques may be used to join DNA segments in vitro. Blunt ends (fully base-paired DNA duplexes) produced by certain restriction endonucleases may be directly ligated with T4 DNA ligase. More usually, DNA segments are linked through their single-stranded cohesive ends and covalently closed by a DNA ligase, e.g. T4 DNA ligase. Such single-stranded "cohesive termini" may be formed by cleaving DNA with another class of endonucleases which produce staggered ends (the two strands of the DNA duplex are cleaved at different points at a distance of a few nucleotides). Single strands can also be formed by the addition of nucleotides to blunt ends or staggered ends using terminal transferase ("homopolymeric tailing") or by simply chewing back one strand of a blunt-ended DNA segment with a suitable exonuclease, such as $\lambda$ exonuclease. A further approach to the production of staggered ends consists in ligating to the blunt-ended DNA segment a chemically synthesized linker DNA which contains a recognition site for a staggered-end forming endonuclease and digesting the resulting DNA with the respective endonuclease.

The components of the hybrid vector according to the invention, such as the yeast promoter, structural gene for the mutant t-PA optionally including a signal sequence, transcription terminator, the replication system etc., are linked together in a predetermined order to assure proper function. The components are linked through common restriction sites or by means of synthetic linker molecules to assure proper orientation and order of the components.

A linear DNA vector is made by methods known in the art, e.g. linking the above yeast promoter to the mutant t-PA coding region in such a manner that the mutant t-PA coding region is controlled by said promoter, and providing the resulting DNA with a DNA segment containing transcription termination signals derived from a yeast gene.

Joining of the DNA segments may be done as detailed above, viz. by blunt end ligation, through cohesive termini or through chemically synthesised linker DNAs.

In particular, in order to be efficiently expressed, the mutant t-PA gene must be properly located with respect to sequences containing transcriptional (yeast promoter) and translational functions (ribosome binding sites). Firstly, the ligation of the DNA segment comprising the promoter with the mutant t-PA coding region has to be achieved in the proper orientation. If two orientations are possible the correct one is determined by conventional restriction analysis. Hybrid vectors containing an incorrectly oriented mutant t-PA gene insert are re-oriented by excising the gene insert with a suitable restriction endonuclease and re-ligating the gene with the hybrid vector fragment. In any case improper orientation is avoided by ligating two DNA segments each with different restriction sites at their ends. Furthermore, the construction of the hybrid vector should be done in such a way that it allows correct transcription initiation and termination. As to the latter point, the transcript should preferably end in a DNA sequence derived from yeast chromosomal DNA or yeast $2\mu$ plasmid. Secondly, a proper reading frame must be established. Ordinarily, the nucleotide sequence of the promoter region and the mutant t-PA coding region is known prior to ligation or can easily be determined so that there are no problems in establishing the correct reading frame.

If the expression of the mature mutant t-PA for accumulation in the cytoplasm is desired, signal sequences or parts thereof optionally following the promoter region and/or optionally preceding the mature mutant t-PA coding region have to be eliminated, for example by digestion with an exonuclease, e.g. with Bal31. A preferred region for directly joining a yeast promoter to the mutant t-PA coding sequence is between the major mRNA start and the ATG of the gene naturally linked to the yeast promoter. For a junction in this region the mutant t-PA coding sequence should have its own ATG for translation initiation, or else it has to be provided with an additional synthetic oligonucleotide. The yeast promoter may also be linked to the mutant t-PA coding sequence by means of a synthetic oligodeoxyribonucleotide as a connecting molecule. Thus, the promoter region may be, if possible, restricted near its 3'-terminus so that it lacks a predetermined number of base pairs. Analogously, the mutant t-PA coding sequence may be restricted near its 5'-terminus. A synthetic oligodeoxynucleotide can then be constructed in such a way that, when joining the yeast promoter and the mutant t-PA coding sequence via the connecting oligodeoxynucleotide, the missing base pairs are restored including an ATG translation initiation signal and the mutant t-PA coding sequence is in the proper reading frame relative to the ATG initiation codon.

4. Transformation of host cells with hybrid vectors containing a mutant t-PA coding sequence

Another aspect of the invention involves transformed eukaryotic host cells containing a DNA sequence coding for a mutant t-PA protein according to the invention.

Suitable eukaryotic host cells are especially those specified above, in particular yeasts including species of the genera Kluyveromyces, Candida, Rhodotorula, Saccharomyces, Schizosaccharomyces, Torulopsis and related genera (cf. J. Lodder, The Yeasts, Amsterdam 1971), especially strains of Saccharomyces cerevisiae.

The transformed eukaryotic host cells are selected from a host cell transformed with a hybrid plasmid comprising a promoter and a DNA sequence coding for a human mutant t-PA protein according to the invention, which DNA sequence is under transcriptional control of said promoter, and a host cell with said DNA sequence stably integrated in a host chromosome and being under the transcriptional control of a chromosomal host promoter.

The process for the production of said transformed eukaryotic host cells comprises transforming eukaryotic host cells with a hybrid vector comprising a promoter and a DNA sequence coding for a human mutant t-PA protein according to the invention, which DNA sequence is under transcriptional control of said promoter.

The transformation of the eukaryotic host cells is accomplished by methods known in the art. For example, the transformation of yeast with the hybrid vectors may be accomplished according to the method described by Hinnen et al [Proc. Natl. Acad. Sci. USA 75, 1929(1978)]. This method can be divided into three steps:

(1) Removal of the yeast cell wall or parts thereof.

(2) Treatment of the "naked" yeast cells (spheroplasts) with the transforming DNA in the presence of PEG (polyethyleneglycol) and $Ca^{2+}$ ions.

(3) Regeneration of the cell wall and selection of the transformed cells in a solid layer of agar.

Preferred methods:

ad (1): The yeast cell wall is removed enzymatically using various preparations of glucosidases, such as snail gut juices (e.g. Glusulase® or Helicase®) or enzyme mixtures obtained from microorganisms (e.g. Zymolyase®) in osmotically stabilized solutions (e.g. 1 M sorbitol).

ad (2): The yeast spheroplasts aggregate in the presence of PEG and local fusions of the cytoplasmic membranes are induced. The generation of "fusion-like" conditions is crucial and many transformed yeast cells become diploid or even triploid during the process of transformation. Procedures which allow selection of fused spheroplasts can be used to enrich for transformants, i.e. transformed cells can easily be screened for among preselected fusion products.

ad (3): Since yeast cells without cell wall do not divide the cell wall has to be regenerated. This regeneration is conveniently done by embedding the spheroplasts into agar. For example, molten agar (about 50°C) is mixed with the spheroplasts. Upon cooling the solution to yeast growth temperatures (about 30°C), a solid layer is obtained. This agar layer is to prevent rapid diffusion and loss of essential macromolecules from the spheroplasts and thereby facilitates regeneration of the cell wall. However, cell wall regeneration may also be obtained (although at lower efficiency) by plating the spheroplasts onto the surface of preformed agar layers.

Preferably, the regeneration agar is prepared in a way to allow regeneration and selection of transformed cells at the same time. Since yeast genes coding for enzymes of amino acid biosynthetic pathways are generally used as selective markers (supra), the regeneration is preferably performed in yeast minimal medium agar. If very high efficiencies of regeneration are required the following two step procedure is advantageous: (1) regeneration of the cell wall in a rich complex medium, and (2) selection of the transformed cells by replica plating the cell layer onto selective agar plates.

If the hybrid vector does not contain any marker gene the transformed cells can also be identified by means of alternative methods. Such methods include, for example, in situ hybridization with a labeled DNA fragment homologous to sequences of the hybrid vector [e.g. according to Hinnen et al., supra], in situ immunoassays provided that an antibody for the product of the introduced gene is available, or other screening methods which measure gene products encoded by the transforming plasmid(s).

Alternatively, the yeast can be co-transformed with a hybrid vector according to the invention and a second vector containing a genetic marker for yeast. If the two different vectors have DNA sequences in common (these can be bacterial sequences present on the vectors), recombination takes place leading to a fused selectable hybrid molecule.

The yeast can also be cotransformed with a linear DNA vector consisting of the yeast promoter, the mutant t-PA coding region optionally including a signal sequence controlled by said promoter and transcription termination signals of a yeast gene, and a vector containing a selective marker for yeast. Cotransformation allows enrichment for those yeast cells which have taken up DNA that cannot be directly selected for. Since competent cells take up any type of DNA a high percentage of cells transformed with a selective vector will also harbor any additional DNA (such as the above linear DNA vector). By virtue of sufficient long homologous sequences (e.g. about 20 to 100 deoxynucleotides in length) the polypeptide gene will be stably integrated into the host chromosome.

The transformed eukaryotic host cells, especially the transformed yeast strains, containing the hybrid plasmids according to the invention or having the linear DNA vector comprising the mutant t-PA gene stably integrated in a host chromosome can be improved in production of mutant t-PA by mutation and selection using methods known in the art. The mutation can be effected, for example, by U.V. irradiation or suitable chemical reagents. Especially preferred is the production of protease deficient mutants, especially yeast mutants, so as to avoid proteolytic degradation of the produced mutant t-PA within the cells.

5. Pharmaceutical preparations

The novel mutant t-PA proteins which are preferably in the one-chain form, obtainable according to the present invention, exhibit valuable pharmacological properties. They can be used in analogy to known plasminogen activators in humans for the prevention or treatment of thrombosis or other conditions where it is desired to produce local fibrinolytic or proteolytic activity via the mechanism of plasminogen activation, such as arteriosclerosis, myocardial and cerebral infarction, venous thrombosis, thromboembolism, post-surgical thrombosis, thrombophlebitis and diabetic vasculopathies.

It has surprisingly been found that the novel mutant t-PA proteins according to the present invention have biological activities which are comparable to or better than those of fully glycosylated natural t-PA. Thus, the novel t-PA mutants are fibrinolytically active. The unique fibrin-directed properties, i.e. the ability to activate plasminogen only in the presence of fibrin, are fully retained. Furthermore, the novel proteins have a prolonged in vivo stability as compared to authentic t-PA. Apparently, the novel proteins which are partially or totally deficient in N-linked carbohydrate chains are less susceptible to proteolytic degradation than the fully glycosylated product.

The retention of biological activity of glycoproteins void of carbohydrate residues is unpredictable and variable. In some instances, deglycosylation or even partial deglycosylation led to impairment or loss of biological activity [D.R. Karp et al., J. Biol. Chem. 257, 7330 (1982); H.T. Kentmans et al., Biochemistry 22, 3067 (1983); H.R. Gradnick et al., Proc. Natl. Acad. Sci. USA 80, 2771/1983)]. The surprising properties of the mutant t-PAs according to the invention are not deducible from former results obtained from chemically or enzymatically deglycosylated t-PA (S.P. Little et al., supra; PCT 84/1960, supra) since chemical or enzymatic treatment of t-PA obviously does not entail a product in which the N-linked carbohydrate chains are totally absent or at least some of the N-linked carbohydrate chains are missing.

The invention relates also to pharmaceutical preparations that contain a therapeutically effective amount of the active ingredient (one-chain or two-chain mutant t-PA or mixtures thereof, preferred is the one-chain form) together with organic or inorganic, solid or liquid pharmaceutically acceptable carriers that are suitable for parenteral, i.e. intramuscular, subcutaneous or intraperitoneal, administration and that do not deleteriously interact with the active ingredients.

There are suitable infusion solutions, preferably aqueous solutions or suspensions, it being possible to prepare these before use, for example from lyophilised preparations that contain the active ingredient alone or together with a carrier, such as mannitol, lactose, glucose, albumin and the like. The pharmaceutical preparations are sterilized and, if desired, mixed with adjuncts, for example preservatives, stabilisers, emulsifiers, solubilisers, buffers and/or salts for regulating the osmotic pressure. Sterilization can be achieved by sterile filtration through filters of small pore size (0.45 $\mu$m diameter or smaller) after which the preparation can be lyophilised, if desired. Antibiotics may also be added in order to assist in preserving sterility.

The pharmaceutical preparations according to the present invention are dispensed in unit dosage forms, for example ampoules, comprising 1 to 2000 mg of a pharmaceutically acceptable carrier per unit dosage and about 1 to 100 mg, preferably about 3 to 25 mg, of the active ingredient (one-chain or two-chain mutant t-PA or mixtures thereof, preferred is the-one-chain form), per unit dosage.

Depending upon the type of the disease and the age and the condition of the patient, the daily dose to be administered for the treatment of a patient weighing approximately 70 kg is in the range from 3 to 100 mg, preferably from 5 to 50 mg, per 24 hours. In the case of myocardial infarction preferably a dose of about 30 to 80 mg is administered within 60 to 120 minutes, preferably in three aliquots and within about 90 minutes.

The invention also provides a method for producing a pharmaceutical preparation characterised in that a biologically active protein according to the present invention is admixed with a pharmaceutically acceptable carrier.

The use of the new proteins for the prophylactic and therapeutic treatment of the human body is also an object of the present invention.

The invention concerns especially the DNA sequences, the hybrid vectors, the transformed host strains, the mutant t-PA proteins and the processes for the preparation thereof as described in the Examples.

Brief description of the drawings

In the following experimental part various embodiments of the present invention are described with reference to the accompanying drawings in which:

Figure 1 provides the DNA and corresponding amino acid sequences of t-PA DNA from HeLa cells. The glycosylation sites are underlined. The arrow indicates the presumable cleavage site between Arg and Ile which generates the two-chain form of t-PA from the one-chain form.

Figure 2 provides the DNA sequences of the mutated t-PA genes and the corresponding amino acid sequences of the mutant t-PA proteins according to the invention.

Figure 3 is a diagram showing the clot lysis activity of mutant t-PAs, viz. [Asn$^{119}$]-t-PA (straight line 3), [Asn$^{119}$, Ala$^{186}$]-t-PA (straight line 4) and [Asn$^{119}$, Ala$^{186}$, Asn$^{450}$]-t-PA, (straight line 5) compared to authentic human melanoma t-PA (straight line 1) and yeast wild-type t-PA (straight line 2).

Figure 4 is a schematic diagram showing the immunodetection of mutant t-PAs by Western blots.

The following Examples serve to illustrate the present invention but should not be construed as a limitation thereof.

Experimental part

The following abbreviations are used in the Examples:

BSA: bovine serum albumin
DTT: 1.4-dithiothreitol (1.4-dimercapto-2.3-butanediol)
EDTA: ethylenediaminetetraacetic acid
PEG: polyethylene glycol 6000
SDS: sodium dodecyl sulphate
SSC: solution containing 150 mM NaCl, 15 mM sodium citrate, 0.5 mM EDTA, adjusted to pH 7.2 with HCl
TBE: solution containing 90 mM Tris, 90 mM boric acid, 2.5 mM Titriplex®
TE: solution containing 10 mM Tris.HCl (pH 8.0) and 0.1 mM EDTA (pH 8.0)
TNE: solution containing 100 mM NaCl, 10 mM Tris.HCl (pH 7.5) and 1 mM EDTA
Tris.HCl: tris-(hydroxymethyl)-aminomethane, pH adjusted with HCl

## Example 1: Cloning of a p31/PHO5-TPA18 BamHI-HindIII fragment containing the tissue plasminogen activator gene into M13mp8

a) The plasmid p31/PHO5-TPA18 (as described in European Patent Application No. 143,081) has the PHO5 promoter and the PHO5 signal sequence fused in frame to the t-PA structural gene (cf. Figure 1). The plasmid is passed through DH-1 (F$^{\ominus}$, recA1, endA1, gyrA96, thi-1, hsdR17 (r$_k^{\ominus}$, m$_k^{\oplus}$), supE44, λ$^{\ominus}$), a dam$^{\ominus}$ strain of E.coli. Two amp$^R$ colonies are isolated and grown in 100 ml LB medium containing 100 µg/ml of ampicillin. Plasmid DNA is isolated from the cells. Restriction enzyme digests with BamHI and HindIII (Boehringer) show the correct size of the t-PA gene insert, which is confirmed by the restriction pattern of the PstI (Boehringer) digest.

b) Three µg of p31/PHO5-TPA18 from E.coli strain DH-1 is incubated at 37°C for 60 min. with 20 U of HindIII (Boehringer, 20 U/µl) in 50 µl of 10 mM Tris.HCl pH 7.5, 6 mM MgCl$_2$, 50 mM NaCl, 6 mM mercaptoethanol. An aliquot is checked on a 1 % agarose gel in TBE buffer to confirm complete digestion. The digest is incubated at 65°C for 10 min. Then 0.5 µl of 5 M NaCl is added followed by 18 U of BamHI (Boehringer, 18 U/µl). This is incubated at 37°C for 60 min. Analysis of an aliquot on a 1 % agarose gel in TBE buffer shows the 2.3 Kb insert besides the 3.6 Kb vector fragment. The insert is isolated on a 0.8 % preparative agarose gel. The gel slice is transferred to a Micro-Colloidon® tube (Sartorius GmbH), covered with 100 $\mu$l of TE and electroeluted (electrophoresed at 90 mA for 50 min.). The TE solution is collected in a siliconized tube. The DNA is precipitated with 2.5 volumes of absolute ethanol after the addition of 0.1 volume 10xTNE. The DNA pellet is washed with cold 80 % ethanol and dried in vacuum. The DNA is resuspended in 20 $\mu$l of TE (13 fmoles/$\mu$l).

c) Three $\mu$g of M13mp8 (RE form) is cut with HindIII and BamHI, and the 7.3 Kb fragment is isolated on a 0.8 % preparative agarose gel, electroeluted and precipitated as above. The DNA is resuspended in 20 $\mu$l of TE (30.8 fmoles/$\mu$l).

d) 92 fmoles of M13mp8 BamHI-HindIII cut vector and 130 fmoles of BamHI-HindIII t-PA insert are ligated in 20 $\mu$l of 50 mM Tris.HCl pH 7.5, 10 mM MgCl$_2$, 10 mM DTT, 2 mM ATP, 1 $\mu$g Gelatin (Sigma) with 400 U of T4 DNA ligase (New England Bio Labs, 400 U/$\mu$l) for 18 hours at 15°C. After incubation at 65°C for 10 min., a 1 $\mu$l aliquot of the ligation mixture is diluted (1:10 with TE). 2 $\mu$l and 8 $\mu$l of this diluted ligation mixture are used for transformation of E.coli JM101 Ca$^{2+}$ cells according to the manual "M13 Cloning and sequencing handbook" published by Amersham. From the 200 colourless plaques (8 $\mu$l, 1:10 diluted ligation mix.), 25 are picked, and single-stranded and replicative form (RF) DNA are prepared [J. Messing, Methods in Enzymology, 101, 21-78 (1983)]. On analysis of RF-DNA, two clones show the correct size insert (2.3 Kb BamHI-HindIII t-PA gene fragment). These two clones containing the t-PA gene with the PHO5 promoter and terminator are further analysed . The PstI, HindIII-EcoRI, BamHI-EcoRI digests confirm the presence of the 2.3 Kb fragment. One of the clones is used for further constructions and is referred to as p8A-7.

Example 2: Mutation of the first glycosylation site at Asn 117 using single-stranded p8A-7

```
                                           117      119

                                         Asn Ser Ser

                                          5'
coding strand of t-PA   ...TGG AAC AGC AGC GCG TTG GCC 3' ...

                                  3'
mutagenic primer I          C TTG TCG TTG CGC AAC C 5'

                            5'
mutated coding strand   ...TGG AAC AGC AAC GCG TTG GCC 3' ...

                                         Asn Ser Asn
```

All mutagenic and sequencing primers are synthesised using the phosphoramidite method [M.H.Caruthers, in Chemical and Enzymatic Synthesis of Gene Fragments (H.G. Gassen and A. Lang, Eds.) Verlag Chemie, Weinheim, Federal Republic of Germany] on an Applied Biosystems (Model 380B) synthesizer.

The following sequencing primers are synthesized:

Sequencing Primer I:

dGCCCAGCCTGATGGCGT

Sequencing Primer II:

dCCACGGGAGGCAGGAGG

Sequencing Primer III:

dCAGCACGTGGCACCAGG

Sequencing Primer IV:

dTGGCAGGCGTCGTGCAA

Universal M13 sequencing primer:

dGTAAAACGACGGCCAGT

a) Phosphorylation of mutagenic primer:

200 pmoles of the mutagenic primer I are phosphorylated in 20 $\mu$l of 50 mM Tris.HCl pH 7.5, 10 mM MgCl$_2$, 5 mM DTT, 0.1 mM spermidine, 0.1 mM EDTA containing 1 $\mu$l of 10 mM ATP using 8 U of T4 polynucleotide kinase (Boehringer, 8 U/$\mu$l). After incubation at 37°C for 45 min., the reaction is stopped by heating at 65°C for 10 min.

b) Annealing of mutagenic primer I and universal sequencing primer to single-stranded template p8A-7:

0.88 $\mu$g (123 fmoles) of single-stranded template p8A-7 is incubated with 20 pmoles of phosphorylated mutagenic oligodeoxyribonucleotide I (10 pmoles/$\mu$l) and 10 pmoles of universal M13 sequencing primer in 10 $\mu$l of 20 mM Tris.HCl pH 7.5, 10 mM MgCl$_2$, 50 mM NaCl, 1 mM DTT at 55°C for 5 min., then cooled at RT for 5 minutes.

c) Extension-ligation reaction:

To the above annealed mix is added 10 $\mu$l of enzyme-dNTP (dATP, dGTP, dTTP, dCTP) solution containing 1 $\mu$l of buffer [0.2 M Tris.HCl pH 7.5, 0.1 M MgCl$_2$, 0.1 M DTT), 4 $\mu$l 2.5 mM dNTP mixture, 1 $\mu$l 10 mM $\gamma$-ATP, 1.4 $\mu$l T4DNA ligase (Amersham, 2.5 U/$\mu$l), 0.6 $\mu$l Klenow DNA polymerase (BRL, 2.99 U/$\mu$l)]. This is incubated at 15°C for 8 hours. The reaction is stopped by incubating at 65°C for 10 min.

d) transformation of ligation mixture:

The ligation mixture is diluted 1:20 and 1:200 with TE. 1 $\mu$l and 5 $\mu$l of each of these diluted solutions are used to transform 0.2 ml of E.coli JM101 competent cells [J. Messing, Methods in Enzymology, 101, 21-78 (1983)]. Thirty-six plaques are picked, phages are isolated by PEG precipitation and redissolved in 50 $\mu$l TE (0.1-0.16 $\mu$g/$\mu$l).

e) Screening of phages:

2 $\mu$l of phage DNA are spotted on dry nitrocellulose paper (Millipore S.A., Cat.No. HAWP090, pore size 0.45 $\mu$m). After drying in air, the filter is baked at 80°C for 2 hours in vacuum.

The filter is prehybridized with 5 ml of 6x SSC, 10x Denhardt's (0.2 % BSA, 0.2 % polyvinyl pyrrolidone, 0.2 % Ficoll), 0.2 % SDS at 67°C for one hour in a heat-sealable bag.

The filter is removed and rinsed in 50 ml of 6x SSC at room temperature for 1 min. The filter is placed in a sealable bag. Three ml of 6x SSC, 10x Denhardt's and 100 $\mu$l of $^{32}$P-labelled mutagenic primer I (5x10$^6$ cpm) are added.

The bag is kept at room temperature for one hour. The filter is removed and washed with 6xSSC (50 ml x 3) for a total of 10 min. at room temperature. The filter is autoradiographed for one hour at -70°C with intensifying screen. The filter is washed at 50°C with 6x SSC (50 ml) and autoradiographed at -70°C for one hour with intensifying screen.

The filter is washed at 60°C with 6x SSC (50 ml) and autoradiographed at -70°C for 2 hours with intensifying screens. On the 60°C washed filter five clones are found to have mutations after initial screening.

f) Sequencing:

Mutation of the AGC codon (Ser119) to the AAC codon (Asn119) is confirmed for one positive clone by DNA sequence determination with sequencing primer I using the chain terminator method [F. Sanger, S. Nickler and A.R. Coulson, Proc. Natl. Acad. Sci. USA 74, 5463-5467(1977)]. The mutation in the DNA results in a Ser→Asn change in amino acid position 119 of mature t-PA ([Asn119]-t-PA).

g) Plaque purifiation:

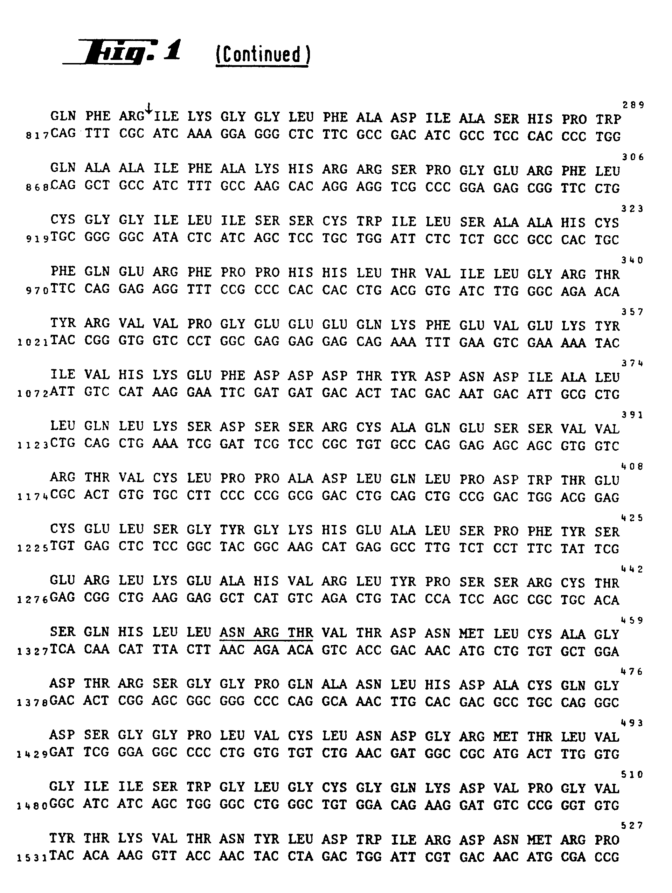

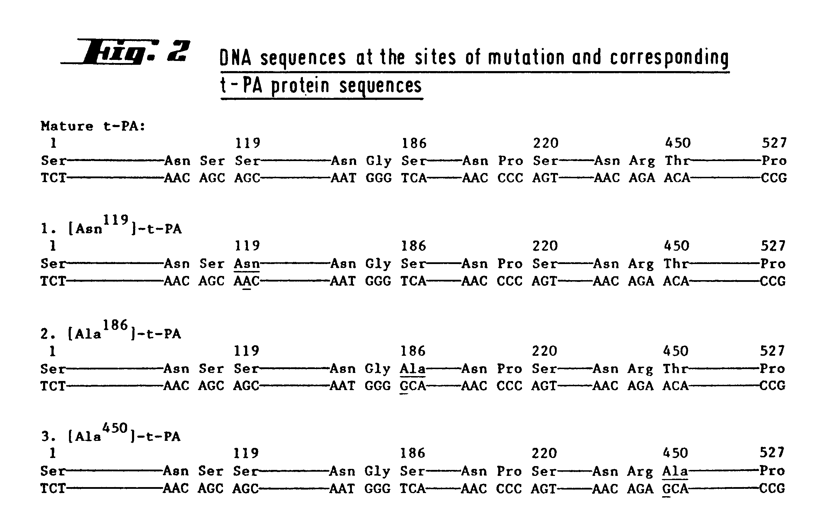

The positive clone designated p8A-7-MO1 is plaque purified. The phage stock is diluted (1:10^7, 10^8, 10^9), plated on YT plates and incubated overnight at 37°C. Then plaques are picked, phages isolated and then screened with 32P-labelled mutagenic primer I . DNA from 7 out of the 10 phages has the desired mutation (cf. Figure 2). From one of these, p8A-7-MO1, RF-DNA is prepared and used for cloning in pJDB207.

Example 3: Mutation of the second glycosylation site at Asn 184 using single-stranded p8A-7.

```
                                 184       186

                               Asn Gly Ser
                              5'              3'
      coding strand         ...GGG AAT GGG TCA GCC TAC CGT ...
                              3'              5'
      mutagenic primer II       CC TTA CCC CGT CGG ATG
                              5'              3'
      mutated coding strand  ...GGG AAT GGG GCA GCC TAC CGT ...

                               Asn Gly Ala
```

Phosphorylation of primer II, annealing, extension-ligation and transformation are done exactly as in Example 2. Thirty-six plaques are picked, and phages screened with 32P-labelled mutagenic primer II. The temperatures at which the filter is washed are: room temperature, 50°C and 60°C. The 60°C wash shows that there are four mutagenic clones. The DNA from one clone is sequenced with sequencing primer II confirming mutation at the second glycosylation site (cf. Figure 2). Phage from this clone is plaque purified and screened for positive mutations. One of the positive clones is referred to as p8A-7-MO2. The mutation converts the codon TCA (Ser186) to GCA (Ala186) resulting in a Ser→Ala change at position 186 of mature t-PA ([Ala186]-t-PA).

Example 4: Mutation of the second glycosylation site at Asn 184 using single-stranded mutated p8A-7-MO1.

The procedure in Example 2 is repeated using single-stranded p8A-7-MO1 and mutagenic primer II. After plaque-purification and screening one of the positive clones is referred to as p8A-7-MO21. The DNA of the clone is sequenced with sequencing primers I and II. Mutation at glycosylation sites 117 and 184 is confirmed (cf. Figure 2). The combination of two mutations in the DNA leads to a mutant t-PA molecule with two amino acid changes at positions 119 and 186 ([Asn119, Ala186]-t-PA).

Example 5: Mutation of the fourth glycosylation site at Asn 448 using single-stranded mutated p8A-7-MO21 (first construct)

```
                                 448       450

                               Asn Arg Thr Val Thr
                              5'              3'
      coding strand         ...CTT AAC AGA ACA GTC ACC GAC A ...
                              3'              5'
      mutagenic primer IV      GAA TTG TCT TTG CAG TGG CTG T
                              5'              3'
      mutated coding strand  ...CTT AAC AGA AAC GTC ACC GAC A ...

                               Asn Arg Asn Val Thr
```

The procedure in Example 2 is repeated using single-stranded p8A-7-MO21 and mutagenic primer IV. The temperatures at which the filter is washed are: room temperature, 50°C, 60°C and 68°C. The 68°C wash identifies the positive clones. One such positive clone after plaque purification is referred to as p8A-7-MO421. The DNA of the clone is subjected to sequence analysis with sequencing primers I, II and IV confirming mutations at glycosylation sites 117, 184 and 448 (cf. Figure 2). With three mutations on the DNA the resulting mutant t-PA molecule has three amino acid exchanges at positions 119, 186 and 450 (-[Asn$^{119}$, Ala$^{186}$, Asn$^{450}$]-t-PA). In this mutant a new glycosylation site has been created at the fourth site by replacing Thr$^{450}$ with Asn.

Example 6: Mutation of the fourth glycosylation site at Asn 448 using single-stranded mutated p8A-7-MO21 (second construct)

```
                                        448       450
                                Leu Leu Asn Arg Thr Val Thr      3'
                        5'
coding strand           ...TCA CAA CAT TTA CTT AAC AGA ACA GTC ACC GAC...

mutagenic primer                            3'                    5'
primer IV-1                                 AA TTG TCT CGT CAG TGG    3'
                        5'
mutated coding          ...TCA CAA CAT TTA CTT AAC AGA GCA GTC ACC GAC...
strand                                      Asn Arg Ala Val Thr
```

The procedure in Example 2 is repeated using single-stranded p8A-7-MO21 and mutagenic primer IV-I. The temperatures at which the filter is washed are: room temperature, 50°C and 60°C in 6xSSC. After plaque purification one of the positive clones is isolated and designated p8A-7-MO421(1). The DNA from the clone is sequened using sequencing primers I, II and IV confirming mutations at glycosylation sites 117, 184 and 448 (cf. Figure 2). With three mutations on the DNA the resulting mutant t-PA molecule has three amino acid exchanges at positions 119, 186 and 450 ([Asn$^{119}$, Ala$^{186}$, Ala$^{450}$]-t-PA).

Example 7: Mutation of the third glycosylation site at Asn 218 using single-stranded mutated p8A-7-MO421-(1)

```
                                218       220
                                Asn Pro Ser
                                5'                    3'
coding strand                   ...G AAC CCC AGT GCC CAG G...
                                3'                    5'
mutagenic primer III             C TTG GGG TTA CGG GTC C
                                5'                    3'
mutated coding strand           ...G AAC CCC AAT GCC CAG G...
                                Asn Pro Asn
```

The procedure in Example 2 is repeated using single-stranded p8A-7-MO421(1) and mutagenic primer III. The temperatures at which the filter is washed are: room temperature, 50°C and 60°C. The 60°C wash identifies the positive clones. After plaque purification one of the positive clones is isolated and designated p8A-7-MO4321(1). The DNA of the clone is sequenced using sequencing primers I to IV. Mutation at glycosylation sites 117, 184, 218 and 448 is confirmed (in Fig. 2). The four mutations on the DNA lead to a mutant t-PA molecule with four amino acid exchanges at positions 119, 186, 220 and 450 ([Asn$^{119}$, Ala$^{186}$, Asn$^{220}$, Ala$^{450}$]-t-PA).

Example 8: Cloning of the mutated t-PA gene inserts into pJDB207

   a) Preparation of RF-DNA:

RF-DNA is prepared for p8A-7-MO1, p8A-7-MO2, p8A-7-MO21, p8A-7-MO421, p8A-7-MO421(1) and p8A-7-MO4321(1) by the quick DNA isolation procedure [D.S. Holmes and M. Quigley, Anal. Biochem. 114, 193-197(1981)]

b) Isolation of inserts:

The six RF-DNAs (0.5 $\mu$g) are digested with 16 U of BamHI and 20 U of HindIII in 50 $\mu$l of 10 mM Tris.HCl pH 7.5, 6 mM MgCl$_2$, 100 mM NaCl, 6 mM mercaptoethanol for one hour at 37°C. After adding 2 $\mu$l of RNase (Serva, 1 mg/ml) and incubating for 5 min. at 37°C, the 2.3 Kb inserts are isolated on a 0.8 % preparative agarose gel. The DNA is extracted by electroelution and after precipitation dissolved in 5 $\mu$l TE (46.8 fmoles/$\mu$l).

c) Three $\mu$g of pJDB207 [J. Beggs in "Genetic Engineering" (ed. R. Williamson), Vol. 2, p. 175-203, Academic Press, New York 1981] are cut with BamHI and HindIII and the 6.6 Kb vector is isolated. After electroelution and precipitation, the DNA is redissolved in 20$\mu$l TE (35 fmoles/$\mu$l).

d) 140 fmoles of pJDB207 BamHI-HindIII cut vector, 234 fmoles of BamHI-HindIII mutated t-PA inserts are ligated in 20 $\mu$l of 50 mM Tris.HCl pH 7.5, 10 mM MgCl$_2$, 10 mM DTT, 2 mM ATP, 1 $\mu$g gelatin with 400 U of T4 DNA ligase for 16 hours at 15°C. The reaction is stopped by incubation at 65°C for 10 min. 2 $\mu$l and 5 $\mu$l of these ligation mixtures are used for transformation of E.coli HB101 Ca$^{\oplus\oplus}$ cells [M. Dagert and S.D. Ehrlich, Gene 6, 23-28 (1979)]. 6 amp$^R$ colonies are picked from each of the six ligations. DNA is prepared by the quick isolation procedure. On analysis of DNA with BamHI-HindIII and PstI correct size bands are observed. One clone from each of the five ligations is grown in 100 ml LB medium containing 100 $\mu$g/ml of ampicillin. Plasmid DNAs derived from p8A-7-MO1, p8A-7-MO2, p8A-7-MO21, p8A-7-MO421, p8A-7-MO421(1) and p8A-7-MO4321(1) are isolated and are referred to as pJDB207/PHO5-TPA18-MO1, pJDB207/PHO5-TPA18-MO2, pJDB207/PHO5-TPA18-MO21, pJDB207/PHO5-TPA18-MO421, pJDB207/PHO5-TPA18-MO421(1) and pJDB207/PHO5 TPA18-MO4321(1).

Example 9: Construction of a yeast expression vector containing the PHO5 promoter, the invertase signal sequence and the tPA coding region

A) Synthesis of oligodeoxyribonucleotides for invertase signal sequence:

Four oligodeoxyribonuclotides: I-1, I-2, I-3, I-4 are synthesized by DNA synthesizer (model 380B Applied Biosystems). After deblocking, the synthetic fragments are purified on a 12 % polyacrylamide gel containing 8 M urea. Salt-free pure oligodeoxyribonucleotides are obtained using Sep. Pak (Waters Associates). These fragments constitute a duplex which encodes the invertase signal sequence with the frequently used yeast codons.

```
         EcoRI              HindIII
                   MetLeuLeuGlnAlaPheLeuPheLeuLeu
     I-1 5'AATTCATGCTTTTGCAAGCTTTCCTTTTCCTTTT 3'
     I-2    3'GTACGAAAACGTTCGAAAGGAAAAGGAAAACCGAC 5'


         AlaGlyPheAlaAlaLysIleSerAla
     I-3 5'GGCTGGTTTTGCAGCCAAAATATCTGCATCTTAGCGTC 3'
     I-4    3'CAAAACGTCGGTTTTATAGACGTAGAATCGCAGAGCT 5'
                                      HgaI
                                        XhoI
```

B) Subcloning of the invertase signal sequence in plasmid p31

a) Preparation of vector:

1.5 $\mu$g of p31R/SS-TPA$\Delta$2 [See Europ. Patent Appl. 143,081] is digested with 10 U of EcoRI (Boehringer) in 50 $\mu$l of 10 mM Tris.HCl pH 7.5, 6 mM MgCl$_2$, 100 mM NaCl, 6 mM mercaptoethanol for one hour at 37°C. After adding 1 $\mu$l of 2.5 M NaCl, 10 U of XhoI (Boehringer) are added and incubated at 37°C for one hour. The 4.2 kb vector is isolated on a 0.8 % preparative agarose gel. The gel slice is transferred to a Micro Colloidon tube (Sartorius GmbH), covered with 200 $\mu$l of TE and electroeluted (electrophoresed at 90 mA for 50 min). The TE solution is collected and precipitated in 2.5 volumes of absolute ethanol after the addition of 0.1 volume 10 x TNE. The DNA pellet is washed with cold 80 % ethanol and dried in vacuum. The DNA is resuspended in 6 $\mu$l TE (40 pmoles/$\mu$l).

b) Annealing oligodeoxyribonucleotides (I-1, I-2, I-3, I-4), kination and ligation with vector

A solution containing 10 pmoles of each of the four deoxyribonucleotides in 10 $\mu$l of 0.5 M Tris.HCl pH 8 is incubated at 95°C for 5 minutes on a water bath. The water bath is slowly cooled to 30°C over a period of 5 hours. To this annealed mixture is added 2 $\mu$l each of 0.1 M MgCl$_2$ 0.1 M NaCl, 30 mnM DTT, 4 mM ATP and 8 U (1 $\mu$l) of polynucleotide kinase (Boehringer). Kination is carried out at 37°C for one hour. The annealed, kinased oligodeoxyribonucleotides and 60 pmoles of p31R/SS-TPAΔ2 EcoRI-XhoI cut vector (1.5 $\mu$l) are ligated with 400 U (1 $\mu$l) of T4 DNA ligase (Biolabs) at 14°C for 17 hours. The reaction is stopped by incubation at 65°C for 10 min. 10 $\mu$l of this ligation mixture is used for transformation of E.coli HB101 Ca$^{++}$ cells [M. Dagert and S.D. Ehrlich, Gene 56, 23-28 (1979)]. 20 amp$^R$ colonies are picked. DNA is prepared by the quick isolation procedure (D.S. Holmes and M. Quigley. Anal. Biochem. 114, 193-197 (1981)]. DNA is digested with EcoRI and XhoI, radio-labelled at the EcoRI end and analysed on a 6 % polyacryalmide gel containing 8 M urea using radiolabelled pBR322 HaeIII cut DNA as marker. Correct size bands are observed for DNA obtained from all the 20 clones. One clone is grown in 100 ml LB medium containing 100 $\mu$g/ml of ampicillin. Plasmid DNA is isolated and is referred to as p31RIT-12.

C) Construction of pJDB207/PHO5-I-TPA

a) Preparation of vector:

Three $\mu$g of pJDB207/PHO5-TPA18 (European Patent Application No. 143,081) is incubated at 37°C for one hour with 10 U of BamHI in 50 $\mu$l of 10 mM Tris.HCl pH 7.5, 6 mM MgCl$_2$, 100 mM NaCl, 6 mM mercaptoethanol. An aliquot is checked on a 1 % agarose gel in TBE buffer to confirm complete digestion. The digest is incubated at 65°C for 10 min. Then 0.5 $\mu$l of 5 M NaCl is added followed by 15 U of XhoI (Boehringer). This is incubated at 37°C for one hour. The 6.8 kb vector is isolated on a 0.8 % preparative agarose gel. The DNA is extracted by electroelution and after precipitation dissolved in TE.

b) XhoI digest of p31/PHO5-TPA18:

Thirty $\mu$g of p31/PHO5-TPA18 (European Patent Application No. 143,081) are incubated at 37°C for one hour with 60 U of XhoI (15 U/$\mu$l) in 200 $\mu$l of 10 mM Tris.HCl pH 8, 6 mM MgCl$_2$, 150 mM NaCl, 6 mM mercaptoethanol, extracted with an equal volume of phenol-chloroform, and precipitated in ethanol.

c) Partial PstI digest of XhoI cut p31/PHO5-TPA18

The precipitated XhoI cut p31/PHO5-TPA18 DNA is resuspended in 250 $\mu$l of 10 mM Tris HCl pH 7.5, 6 mM MgCl$_2$, 50 mM NaCl, 6 mM mercaptoethanol, 2.5 mg ethidium bromide, incubated at 37°C for 35 minutes with 22.5 U of PstI, and extracted with an equal volume of phenol, followed by an equal volume of chloroform-isoamylalcohol (50:1). The 1.6 kb fragment is isolated on a 1 % preparative agarose gel. The DNA is extracted by electroelution and precipitated [insert 1].

d) SalI-XhoI digest of p31RIT-12:

Thirty $\mu$g p31RIT-12 are incubated at 37°C for one hour with 60 U of SalI (Boehringer 12 U/$\mu$l) and 60 U of XhoI (15 U/$\mu$l) in 200 $\mu$l of 10 mM Tris.HCl pH 8, 6 mM MgCl$_2$, 150 mM NaCl, 6 mM mercaptoethanol, extracted with an equal volume of phenol-chloroform and precipitated in ethanol. The 869 bp fragment is isolated on a 1.2 % preparative agarose gel. The DNA is extracted by electroelution, desalted over DE-52, and precipitated in ethanol.

e) HgaI digest of SalI-XhoI cut p31RIT-12

SalI-XhoI cut p31RIT-12 is resuspended in 100 $\mu$l of 6 mM Tris.HCl pH 7.5, 10 mM MgCl$_2$, 50 mM NaCl, 1 mM dithiothreitol, 10 mg bovine serum albumin and is incubated at 37°C for one hour with 6 U of

Hgal (Biolabs, 0.5 U/$\mu$l) The 600 bp fragment is isolated on a 1.2 % agarose gel. The DNA is extracted by electroelution and precipitated in ethanol.

f) Annealing of linker oligonucleotides

90 pmoles of two oligodeoxyribonucleotides having the sequences

```
                                          PstI
              5' CTGCATCTTACCAAGTGATCTGCA 3'
                 3'AGAATGGTTCACTAG 5'
```

are suspended in 10 $\mu$l of 0.5 mM Tris.HCl pH 8 in a siliconized Eppendorf tube. The solution is incubated at 95°C for 5 min and then slowly cooled to room temperature overnight.

g) Kination of linker

To the above solution is added 2 $\mu$l of 0.1 M KCl, 2 $\mu$l of 0.1 M MgCl$_2$, 3$\mu$l of 30 mM DTT, 1 $\mu$l of 200 mM ATP, 8 U of polynucleotide kinase (80/$\mu$l). This is incubated at 37°C for one hour.

h) Ligation of the Hgal fragment from p31RIT-12 with the kinased linker

The kinased linker solution is transferred to a tube containing the dry Hgal fragment, and 400 U of T$_4$ DNA ligase is then added. The solution is incubated at room temperature (21-22°C) for 90 minutes, diluted to 100 $\mu$l with TE and extracted with an equal volume of phenol-chloroform. The fragment is precipitated by adding 0.6 volume of isopropanol and 0.1 volume of 3 M sodium acetate at room temperature to the aqueous solution.

i) BamHI-PstI digest of above

The above dry DNA is digested with 10 U of BamHI and 10 U of PstI in 20 $\mu$l of 10 mM Tris.HCl pH 7.5, 100 mM MgCl$_2$, 6 mM mercaptoethanol for one hour at 37°C. After dilution to 100 $\mu$l the solution is extracted with an equal volume of phenol-chloroform, and the aqueous layer is precipitated in isopropanol [insert 2].

j) Ligation of the three fragments

100 pmoles of pJDB207/PHO5-TPA18 BamHI-XhoI cut vector fragment, 200 pmoles of each of the other two insert fragments [1 and 2] are ligated in 10 $\mu$l of 50 mM Tris.HCl pH 7.5, 10 mM MgCl$_2$, 10 mM DTT, 2 mM ATP, 0.5 $\mu$g gelatin with 400 U of T$_4$ DNA ligase for 16 hours at 15°C. The reaction is stopped by incubation at 65°C for 10 min. 5 $\mu$l of this ligation mixture is used for transformation of E.coli HB101 Ca$^{++}$ cells. 10 amp$^R$ colonies are picked and DNA is prepared by the quick isolation procedure. On analysis with EcoRI, PstI and BamHI-HindIII correct size fragments are observed. One clone is grown in 100 ml of LB medium containing 100 $\mu$g/ml of ampicillin. Plasmid DNA is isolated and is referred to as pJDB207/PHO5-I-TPA.

**Example 10: Construction of a yeast expression vector containing the _PHO5_ promoter, the _PHO5_ signal sequence or the invertase signal sequence and the tPA coding region with only the fourth glycosylation site removed.**

The BamHI-HindIII vector fragment of plasmid pJDB207 is ligated with a BamHI-ScaI fragment obtained from pJDB207/PHO5-I-TPA or pJDB207/PHO5-TPA18 and a ScaI-HindIII fragment obtained from pJDB207/PHO5-TPA18-MO421(1).

a) Preparation of vector:

Three μg of pJDB207 are cut with BamHI and HindIII and the 6.6 Kb vector is isolated. After electroelution, the DNA is precipitated in ethanol.

b) ScaI-HindIII digest of pJDB207/PHO5-TPA18-MO421(1):

1.5 μg of the plasmid DNA are digested with 7 U of ScaI (Boehringer) and 6 U of HindIII in 20 μl of 10 mM Tris-HCl pH 7.5, 6 mM MgCl$_2$, 50 mM NaCl, 6 mM mercaptoethanol for one hour at 37°C. After phenolization and precipitation in ethanol the 987 bp fragment is isolated on a 1.2 % preparative agarose gel (insert 1).

c) BamHI-ScaI digest of pJDB207/PHO5-TPA18 and pJDB207/PHO5-I-TPA:

1.5 μg of the two plasmid DNAs are digested with 7 U of BamHI and 7 U of ScaI in 20 μl of 10 mM Tris.HCl pH 7.5, 6 mM MgCl$_2$, 6 mM mercaptoethanol for one hour at 37°C. After phenolization and precipitation in ethanol the 1300 bp fragments are isolated on a 1.2 % preparative agarose gel (insert 2).

d) Ligation of the three fragments:

100 pmoles of pJDB207/PHO5-TPA18 BamHI-HindIII cut vector fragment, 200 pmoles of each of the other two insert fragments [1 and 2] are ligated in 10 μl of 50 mM Tris.HCl pH 7.5, 10 mM MgCl$_2$, 10 mM DTT, 2 mM ATP, 0.5 μg gelatin with 400 U of T$_4$ DNA ligase for 16 hours at 15°C. The reactions are stopped by incubation at 65°C for 10 min. 5 μl of this ligation mixture is used for transformation of E.coli HB101 Ca$^{++}$ cells. 10 amp$^R$ colonies are picked and DNA is prepared by the quick isolation procedure. On analysis with EcoRI, PstI and BamHI-HindIII correct size fragments are observed. One clone from each of the two constructions is grown in 100 ml of LB medium containing 100 μg/ml of ampicillin. Plasmid DNAs are isolated and are referred to as pJDB207/PHO5-TPA18-MO4 and pJDB207/PHO5-I-TPA-MO4.

## Example 11: Cloning of the mutated t-PA gene inserts into a yeast expression vector containing the PHO5 promoter and the invertase signal sequence

The BamHi-HindIII vector fragment of plasmid pJDB207 is ligated with a BamHI-NarI fragment obtained from pJDB207/PHO5-I-TPA and a NarI-HindIII fragment obtained from the mutated t-PA genes.

a) Preparation of RF DNA

RF-DNA is prepared for p8A-7-MO1, p8A-7-MO2, p8A-7-MO21, p8A-7-MO421, p8A-7-MO421(1) and p8A-7-MO4321(1).

b) Isolation of the NarI-HindIII inserts from mutated tPA genes

The six RE-DNAs (1 μg) are each digested with 6 U of NarI (Biolabs) in 20 μl of 10 mM Tris.HCl pH 7.5, 6 mM MgCl$_2$, 6 mM mercaptoethanol at 37°C for one hour. The digest is incubated at 65°C for 10 min., then 1 μl of 1 M NaCl is added followed by 6 U of HindIII. After incubation at 37°C for one hour, RNase (2 μl, 0.5 μg/μl) is added. After 15 min at 37°C and phenolization, DNAs are precipitated in ethanol. The 1450 bp DNA fragments are isolated on a 1.2 % preparative agarose gel. The DNAs are extracted by electroelution and precipitated in ethanol (insert 1).

c) Isolation of the BamHI-NarI insert

Three μg pJDB207/PHO5-I-TPA are digested with 6 U of NarI in 20 μl of 10 mM Tris.HCl pH 7.5, 6 mM MgCl$_2$, 6 mM mercaptoethanol at 37°C for one hour. Then 2 μl of 1 M NaCl is added followed by 6 U of BamHI. After incubation at 37°C for one hour, phenolization, DNA is precipitated in ethanol. The 930 bp DNA fragment is isolated on a 1.2 % preparative agarose gel. The DNA is extracted by electroelution and precipitated in ethanol (insert 2).

d) Preparation of vector

Three μg of pJDB207 are cut with BamHI and HindIII and the 6.6 Kb vector is isolated. After electroelution the DNA is precipitated in ethanol.

e) Ligation of the three fragments

100 pmoles of pJDB207/PHO5-TPA18 BamHI-HindIII cut vector fragment, 200 pmoles of each of the other two insert fragments [1 and 2] are ligated in 10 μl of 50 mM Tris.HCl pH 7.5, 10 mM MgCl$_2$, 10 mM DTT, 2 mM ATP, 0.5 μg gelatin with 400 U of T$_4$ DNA ligase for 16 hours at 15°C. The reactions are stopped by incubation at 65°C for 10 min. 5 μl of this ligation mixture is used for transformation of E.coli HB101 Ca$^{++}$ cells. 10 amp$^R$ colonies are picked and DNA is prepared by the quick isolation procedure. On analysis with EcoRI, PstI and BamHI-HindIII correct size fragments are observed. One clone from each of the six constructions are grown in 100 ml of LB medium containing 100 µg/ml of ampicillin. Plasmid DNAs are isolated and are referred to as pJDB207/PHO5-I-TPA-MO1, pJDB207/PHO5-I-TPA-MO2, pJDB207/PHO5-I-TPA-MO21, pJDB207/PHO5-I-TPA-MO421, pJDB207/PHO5-I-TPA-MO421(1) and pJDB207/PHO5-I-TPA-MO4321(1).

Example 12: Transformation of Saccharomyces cerevisiae GRF18

The plasmids pJDB207/PHO5-TPA18-MO1, pJDB207/PHO5-TPA18-MO2, pJDB207/PHO5-TPA18-MO4, pJDB207/PHO5-TPA18-MO21, pJDB207/PHO5-TPA18-MO421, pJDB/PHO5-TPA18-421(1), pJDB207/PHO5-TPA18-MO4321(1), pJDB207/PHO5-I-TPA-MO1, pJDB207/PHO5-I-TPA-MO2, pJDB207/PHO5-I-TPA-MO4, pJDB207/PHO5-I-TPA-MO21, pJDB207/PHO5-I-TPA-MO421, pJDB207/PHO5-I-TPA-MO421(1) and pJDB207/PHO5-I-TPA-MO4321(1) are each introduced into Saccharomyces cerevisiae strain GRF18 ($\alpha$, his 3-11, his 3-15, leu 2-3, leu 2-112, can$^R$) using the transformation protocol described by Hinnen et al [Proc. Natl. Acad. Sci. USA 75, 1929(1978)]. Five µg each of plasmid DNA are added to 100 µl of a spheroplast suspension and the mixture is treated with polyethylene glycol. The spheroplasts are mixed with 10 ml regeneration agar and plated onto yeast minimal medium plates without leucine. After incubation for 3 days at 30°C about 200 transformed cells are obtained.

One colony from each of the yeast transformations is picked.

The different colonies are referred to as

Saccharomyces cerevisae GRF18/pJDB207/PHO5-TPA18-MO1
Saccharomyces cerevisae GRF18/pJDB207/PHO5-TPA18-MO2
Saccharomyces cerevisae GRF18/pJDB207/PHO5-TPA18-MO4
Saccharomyces cerevisae GRF18/pJDB207/PHO5-TPA18-MO21
Saccharomyces cerevisae GRF18/pJDB207/PHO5-TPA18-MO421
Saccharomyces cerevisae GRF18/pJDB207/PHO5-TPA18-MO421(1)
Saccharomyces cerevisae GRF18/pJDB207/PHO5-TPA18-MO4321(1)
Saccharomyces cerevisae GRF18/pJDB207/PHO5-I-TPA-MO1
Saccharomyces cerevisae GRF18/pJDB207/PHO5-I-TPA-MO2
Saccharomyces cerevisae GRF18/pJDB207/PHO5-I-TPA-MO4
Saccharomyces cerevisae GRF18/pJDB207/PHO5-I-TPA-MO21
Saccharomyces cerevisae GRF18/pJDB207/PHO5-I-TPA-MO421
Saccharomyces cerevisae GRF18/pJDB207/PHO5-I-TPA-MO421(1)
Saccharomyces cerevisae GRF18/pJDB207/PHO5-I-TPA-MO4321(1)

Example 13: Transformation of Saccharomyces cerevisae strain HT246

S. cerevisiae strain HT246 is obtained by repeated backcrossing of the mutant S. cerevisiae strain HT 232 lacking protease B activity [D.H. Wolf et al., in G.N. Cohen and H. Holzer (editors), Limited Proteolysis in Microorganisms, conference report, Bethesda 1978, p. 61] and S. cerevisiae H423 (European Patent Application No. 143,081).

S. cerevisiae HT246 is transformed with plasmids pJDB207/PHO5-TPA18-MO1, pJDB207/PHO5-TPA18-MO2, pJDB207/PHO5-TPA18-MO4, pJDB207/PHO5-TPA18-MO21, pJDB207/PHO5-TPA18-MO421, pJDB/PHO5-TPA18-421(1), pJDB207/PHO5-TPA18-MO4321(1), pJDB207/PHO5-I-TPA-MO1, pJDB207/PHO5-I-TPA-MO2, pJDB207/PHO5-I-TPA-MO4, pJDB207/PHO5-I-TPA-MO21, pJDB207/PHO5-I-TPA-MO421, pJDB207/PHO5-I-TPA-MO421(1) and pJDB207/PHO5-I-TPA-MO4321(1) using the transformation protocol of Hinnen et al. (supra) selecting for leucine prototrophy (cf. Example 12). One clone each is picked and designated.

Saccharomyces cerevisae HT246/pJDB207/PHO5-TPA18-MO1
Saccharomyces cerevisae HT246/pJDB207/PHO5-TPA18-MO2
Saccharomyces cerevisae HT246/pJDB207/PHO5-TPA18-MO4
Saccharomyces cerevisae HT246/pJDB207/PHO5-TPA18-MO21
Saccharomyces cerevisae HT246/pJDB207/PHO5-TPA18-MO421
Saccharomyces cerevisae HT246/pJDB207/PHO5-TPA18-MO421(1)

Saccharomyces cerevisae HT246/pJDB207/PHO5-TPA18-MO4321(1)
Saccharomyces cerevisae HT246/pJDB207/PHO5-I-TPA-MO1
Saccharomyces cerevisae HT246/pJDB207/PHO5-I-TPA-MO2
Saccharomyces cerevisae HT246/pJDB207/PHO5-I-TPA-MO4
Saccharomyces cerevisae HT246/pJDB207/PHO5-I-TPA-MO21
Saccharomyces cerevisae HT246/pJDB207/PHO5-I-TPA-MO421
Saccharomyces cerevisae HT246/pJDB207/PHO5-I-TPA-MO421(1)
Saccharomyces cerevisae HT246/pJDB207/PHO5-I-TPA-MO4321(1)

Example 14: Preparation of yeast cell extracts and determination of t-PA activity

Yeast cells are grown at 30°C in 20 ml of HE-17 medium (8.4 g Yeast Nitrogen Base (Difco), 10 g L-asparagine, Sigma, 1 g L-histidine (Sigma), 40 ml 50 % glucose per 1 litre solution) in a 50 ml Erlenmeyer flask with shaking for 24 hours until a density of 8-10x$10^7$ cells/ml is reached. The cells are centrifuged, resuspended in 10 ml 0.9 % NaCl. Two ml of the resuspended cells are used to inoculate 50 ml low-$P_i$ minimal medium (as described in European Patent Application No. 143081) to which 10 g/l L-asparagine, Sigma, and 10 g/l L-histidine, Sigma, are added in 250 ml Erlenmeyer flasks. Incubation is at 30°C at 250 rpm.

Cells from 10 ml of low $P_i$ minimal medium are collected after 24 and 48 hours by centrifugation at 3000 rpm for 10 minutes in Falcon 2070 tubes. The cells are washed once with 10 ml low $P_i$ medium and centrifuged. The cell pellet is suspended in lysis buffer [66 mM potassium phosphate pH 7.4, 4 mM Zwittergent (Calbiochem.)]. To the cell suspension are added 8 g of glass beads (0.5-0.75 mm in diameter) and a small glass rod and the suspension is shaken on a Vortex Mixer (Scientific Instruments Inc., USA) at fullspeed for 4x2 min with intervals of 2 min on ice. More than 90 % of the cells are broken by this procedure. Cell debris and glass beads are sedimented by centrifugation for 5 min at 3000 rpm at 4°C. The supernatant is transferred to Eppendorf tubes.

t-PA activity is determined using the colorimetric assay as described by J.H. Verheijen et al. [Thromb. Haemost. 48, 226 (1982)].

The obtained results are summarized in Table 1:

Table 1

| S. cerevisiae strain | I.U./l/OD$_{600}$ | µg/l/OD$_{600}$ |
|---|---|---|
| GRF18/pJDB207/PHO5-TPA18-MO1 | 250 | 9 |
| GRF18/pJDB207/PHO5-TPA18-MO21 | 950 | 34 |
| GRF18/pJDB207/PHO5-TPA18-MO421 | 650 | 23 |
| HT246/pJDB207/PHO5-TPA18-MO1 | 1112 | 40 |
| HT246/pJDB207/PHO5-TPA18-MO21 | 5330 | 190 |
| HT246/pJDB207/PHO5-TPA18-MO421 | 2240 | 80 |
| HT246/pJDB207/PHO5-TPA18 (control) | 1112 | 40 |

Example 15: Fermentation of transformed yeast strains on a 3 l scale

All transformants are maintained at the temperature of liquid N$_2$ (vapour phase) in a medium of the following composition (values in g/l):

| sodium glutamate | 50 |
|---|---|
| sucrose | 50 |
| dextrane | 50 |

For a 3 l scale fermentation of each of the transformed S. cerevisiae strains two precultures are prepared:
One loop of cells of a freshly grown culture on agar slants is inoculated into 50 ml of preculture medium consisting of (g/l)

| yeast nitrogen base (Difco 0919-15) | 8.4 |
|---|---|
| L-asparagine | 10 |
| L-histidine | 1 |
| glucose | 20 |

and incubated at 30°C and 180 r.p.m. for 48 hours. 10% of the first preculture is inoculated into the second preculture and incubated for another 24 hours.

The second preculture is centrifuged, the cells resuspended in 0.9% NaCl and inoculated into 3 l fermentation medium having the following composition (g/l):

| glucose | 20 |
|---|---|
| bacto-peptone | 15 |
| ammonium sulfate | 3 |
| yeast extract, oxoid | 1 |
| L-histidine | 0.1 |
| $MgSO_4$-7 $H_2O$ | 1 |

to give an optical density ($OD_{600}$) of 0.2.

Fermentations are run in a 3 l MBR bioreactor (MBR Bio Reactor AG, Wetzikon, Switzerland) at 30°C, an agitation rate of 500 rev/min and an aeration rate of 150 l/h. After 48 hours an optical density of about 12 is reached. The culture broth is cooled to 4°C, centrifuged and the cell pellet resuspended in 150 ml breakage buffer consisting of 4 mM Zwittergent 9-14 (Calbiochem) in 50 mM phosphate-buffer pH 7.4.

150 g glass beads (diameter 0.5 mm) are added and the cells broken for 30 min, setting 5 on a multi-tube vortexer (Scientific manufacturing industries, USA).

Example 16: Purification of the produced mutant t-PA

The ruptured cell suspension (see Example 14) is centrifuged and to the supernatant is added solid ammonium sulfate until 35 % saturation in ammonium sulfate is reached. The suspension is centrifuged and the pellet solubilized in 0.2 M ammonium acetate containing 0.1 % Synperonic® PE/F 68. 5 g (wet weight) of DE-3 inhibitor coupled to Sepharose®-4B beads are added and the suspension gently agitated at 4°C overnight. The beads are separated from the solution, washed twice with 0.2 M sodium chloride containing 0.1 % Synperonic®, filled in a column of diameter 10 mm, and washed with 10 ml of 0.2 M sodium chloride containing 0.1 % Synperonic®, followed by 10 ml of 0.2 M $NH_4$SCN in 0.2 M ammonium acetate containing 0.1 % Synperonic®. T-PA is than eluted with 1.2 M $NH_4$SCN in 0.2 M ammonium acetate containing 0.1 % Synperonic ® at a flow rate of 30 ml/h.

Fractions with the highest fribinolytic activity are pooled. The pool containing the mutant t-PA at a purity of about 90 % or more is used for the characterization studies and biological assays.

Example 17: Activity of the mutant t-PAs in biological assays

The expression of plasmids pJDB207/PHO5-TPA18-MO1, pJDB207/PHO5-TPA18-MO2, pJDB207/PHO5-TPA18-MO4, pJDB207/PHO5-TPA18-MO21, pJDB207/PHO5-TPA18-MO421, pJDB207/PHO5-TPA18-MO421(1), pJDB207/PHO5-I-TPA-MO1, pJDB207/PHO5-I-TPA-MO2, pJDB207/PHO5-I-TPA-MO4, pJDB207/PHO5-I-TPA-MO21, pJDB207/PHO5-I-TPA-MO421, and pJDB207/PHO5-I-TPA-MO421(1) in the S. cerevisiae strain HT246 results in biologically active mutant t-PAs, namely [Asn$^{119}$]-t-PA, [Ala$^{156}$]-t-PA, [Ala$^{450}$]-t-PA, [Asn$^{119}$, Ala$^{186}$]-t-PA, [Asn$^{119}$, Ala$^{186}$, Asn$^{450}$]-t-PA, and [Asn$^{119}$, Ala$^{186}$, Ala$^{450}$]-t-PA, the activity of which is similar or superior to the activity of yeast wild-type t-PA. All mutant t-PAs show activity on casein plates and fibrin plates [J. Jespersen et al., Haemostasis 18, 301 (1983)], in the colorimetric assay described by J.H. Verheijen et al. [Thromb. Haemost. 48, 266 (1982)] and in a fluorimetric assay using Cbz-Gly-Gly-Arg-AMS as substrate [M. Zimmerman et al., Proc. Natl. Acad. Sci. USA 75, 750 (1978)].

In the fluorometric assay all t-PA solutions are diluted to exactly 20 FU/ml (0.28 $\mu$g/ml) in a 60 mM phosphate buffer pH 7.4 containing 0.01% Tween 80 and 0.05% HSA. It is established that about 90% of all t-PAs is in the one-chain form and about 10% is in the two-chain form.

Tests are performed to determine the clot lysis activity of the different mutant t-PAs, basically according to R.D. Philo and P.J. Gaffney (Thromb. Haemost. 45, 107-109 (1981)).

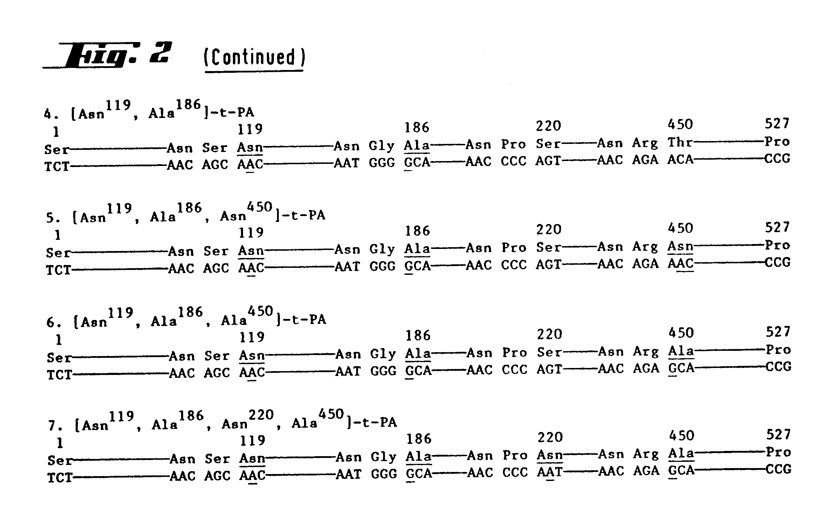

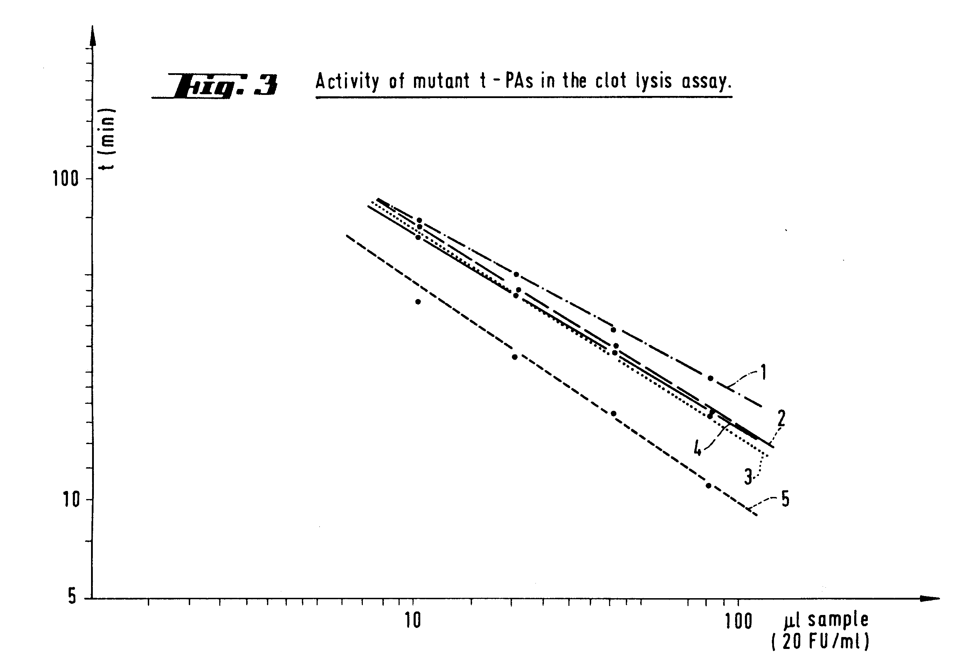

Plotting log of the lysis time versus log of the t-PA concentration results in a straight line, the slope of which is a measure of the fibrin specificity of the plasminogen activator added. By comparison with a standard preparation clot lysis I.U./ml can be determined. The results obtained with the different mutant t-PAs is shown in Fig. 3.

It is seen from the figure that [Asn119]-t-PA (straight line 3 and [Asn119, Ale186]-t-PA (straight line 4) lyse in vitro clots with approximately the same slope as the reference melanoma t-PA (mt-PA, straight line 1). It is concluded that all t-PAs measured have a similar fibrin activity. However, [Asn119, Ala186, Asn450]-t-PA (straight line 5) lyses clots about twice as fast as the melanoma t-PA, the yeast wild-type t-PA (straight line 2, cf. European Patent Application No. 143,081), [Asn119]-t-PA and [Asn119, Ala186]-t-PA.

The ratio of the activities (in I.U./ml) obtained in the above assays is summarised in Table 2.

Table 2

|  | VIU/FU | CIU/FU | CIU/VIU |
|---|---|---|---|
| mt-PA | 4.1 | 3.3 | 1.2 |
| wild type t-PA | 2.5 | 4.7 | 1.9 |
| [Asn119]-t-PA | 3.2 | 5.1 | 1.6 |
| [Asn119, Ala186]-t-PA | 3.8 | 5.4 | 1.4 |
| [Asn119, Ala186, Asn450]-t-PA | 5.4 | 12.1 | 2.2 |
| VIU = I.U. according to Verjeijen et al. (supra) | | | |
| FU = fluorometric units according to Zimmermann et al. (supra) | | | |
| CIU = clot lysis I.U. according to Philo et al. (supra) | | | |

Example 18: Immunodetection

The identity of the clot lysing activity purified from crude yeast extracts with t-PA is shown in Western blots using rabbit polyclonal antibodies directed against melanoma t-PA.

About 12 FU of the different t-PA molecules are separated by polyacrylamide gel electrophoresis using 12.5% gels under reducing conditions.

Protein is then transferred to a nitrocellulose membrane basically according to a manufacturers instruction (Schleicher & Schuell, Feldbach, Switzerland). Transfer is carried out in transfer buffer (g/l: glycine 14.4, Tris 3, methanol 20%) for 2.5 hours at 40 V/cm. After blocking in 3 % BSA for 1 hour, the nitrocellulose is incubated overnight with the antibody (rabbit serum, 100µl to 40 ml BSA-buffer).

Antibody-t-PA complexes are then decorated with $^{125}$J-protein A (4µCi to 40 ml BSA-buffer) for 2 hours.

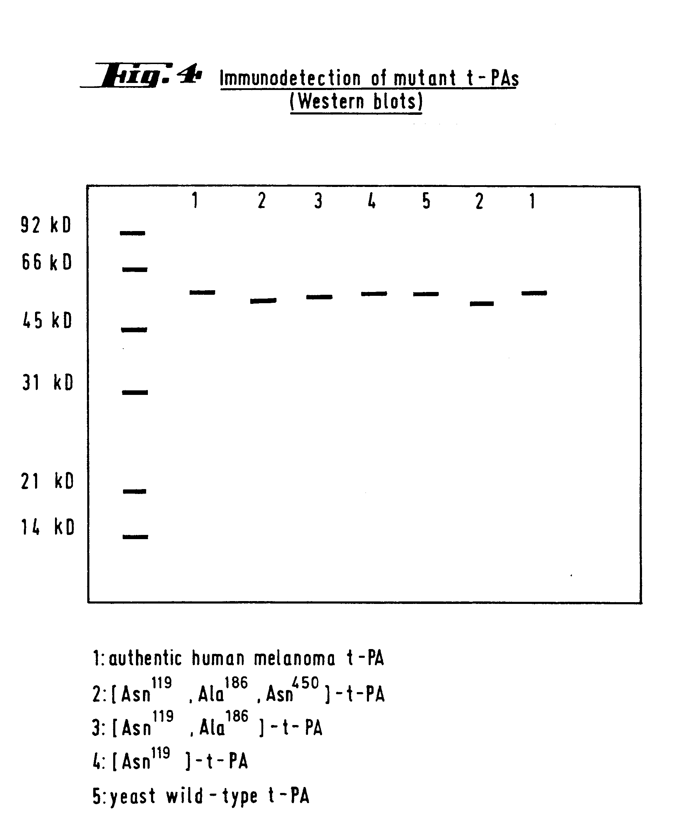

After extensive washes the nitrocellulose membrane is exposed to X-ray film. The result is shown in Figure 4. All mutant t-PAs are detected by the polyclonal antibody and show a reduction in size corresponding to lacking glycolytic side chains. Each mutation accounts for a loss of about 1 kD in molecular weight.

Example 19: Glycosylation state of the mutant t-PAs

Glycosylation is determined with a $^{125}$J-concanavalin A overlay assay after gel electrophoresis. Concanavalin A is a plant lectin which specifically recognizes mannose residues. Again 12 FU of each mutant are applied to 12.5% PAGE. Then the ten proteins are fixed for 30 min in 7% acetic acid. The gel is then washed in lectin buffer having the following composition: 0.15 M NaCl
50.0 mM Tris pH7.4
0.5 mM $CaCl_2$
0.5 mM $MnCl_2$
Washing is continued until the pH reaches about 7.3.

The gel is then carefully overlayered with 2 ml lectin buffer containing 3 mg hemoglobin, 100 µg concanavalin A and 2 µCi $^{125}$J-concanavalin A.

After overnight incubation in a humidified chamber the gel is extensively washed in lectin buffer, dried

26

and exposed to X-ray film. All mutant t-PAs are stained with [125]J-concanavalin A. Since even [Asn[119], Ala[186], Asn[220], Ala[450]]-t-PA slightly reacts with the lectin, it is concluded that this mutant contains residual O-linked sugar moieties.

Example 20: Pharmaceutical preparation for parenteral administration

A solution containing [Asn[119], Ala[186], Ala[450]]-t-PA in the one-chain form, in the two-chain form or mixtures thereof obtained as described above is dialysed against 0.3 molar sodium chloride containing 0.01 % Tween 80® and stored at -80°C. Prior to administration the concentration is adjusted to 75 $\mu$g/ml of total t-PA (i.e. one-chain plus two-chain t-PA) and 0.3M NaCl. The solution is sterilised by filtration through a 0.22 $\mu$m membrane filter.

Instead of the above-mentioned t-PA it is also possible to use the same amount of a different t-PA described in the preceding Examples, such as, for example, [Asn[119]]-t-PA, [Ala[186]]-t-PA, [Ala[450]]-t-PA, [Asn[119], Ala[186]]-t-PA or [Asn[119], Ala[186], Asn[450]]-t-PA.

This procedure is suitable for the preparation of solutions of mutant t-PAs in the one-chain or in the too-chain form or of mixtures thereof for parenteral, such as intravenous, administration.

**Claims**
**Claims for the following Contracting States : BE, DE, FR, GB, IT, NL, SE, CH, LU**

1. Human t-PA proteins having the amino acid sequence

$$A_{1-116}\text{-Asn-Ser-}Y_1\text{-}A_{120-183}\text{-Asn-Gly-}Y_2\text{-}A_{187-217}\text{-Asn-Pro-}Y_3\text{-}A_{221-447}\text{-Asn-Arg-}Y_4\text{-}A_{451-527} \quad \text{(I')}$$

in which $A_{1-116}$ represents the N-terminal amino acid sequence consisting of amino acids 1 to 116 of mature t-PA, $A_{120-183}$ represents the amino acid sequence consisting of amino acids 120 to 183 of mature t-PA, $A_{187-217}$ represents the amino acid sequence consisting of amino acids 187 to 217 of mature t-PA, $A_{221-447}$ represents the amino acid sequence consisting of amino acids 221 to 447 of mature t-PA, $A_{451-527}$ represents the C-terminal amino acid sequence consisting of amino acids 451 to 527 of mature t-PA, and

   a) $Y_1$ and $Y_3$ are Ser, $Y_2$ is a genetically encoded amino acid other than Ser and Thr, and $Y_4$ is Thr; or

   b) $Y_1$, $Y_2$ and $Y_3$ are each Ser and $Y_4$ is a genetically encoded amino acid other than Ser and Thr; or

   c) $Y_1$, $Y_2$ and $Y_4$ are each independently a genetically encoded amino acid other than Ser and Thr, and $Y_3$ is Ser, in the one-chain or in the two-chain form.

2. A t-PA protein according to claim 1 selected from the group consisting of [Ala[186]]-t-PA, [Ala[450]]-t-PA, [Asn[119], Ala[186], Asn[450]]-t-PA and [Asn[119], Ala[186], Ala[450]]-t-PA.

3. [Ala[186]]-t-PA according to claim 1.

4. [Ala[450]]-t-PA according to claim 1.

5. [Asn[119], Ala[186], Ala[450]]-t-PA according to claim 1.

6. [Asn[119], Ala[186], Asn[450]]-t-PA according to claim 1.

7. Human t-PA proteins in the one-chain form according to any one of claims 1 - 6.

8. Process for the preparation of a human t-PA protein according to claim 1, comprising culturing under appropriate nutrient conditions transformed eukaryotic host cells containing a DNA sequence coding for said human t-PA protein, and isolating said human t-PA protein and, if desired, separating a mixture of the one-chain and two-chain forms of the t-PA protein obtained into the individual components, and for the production of the two-chain form which is free of the one-chain form, converting the one-chain form of the t-PA protein produced into the two-chain form.

9. The human t-PA proteins obtainable by the process according to claim 8.

**10.** A DNA having a DNA sequence coding for a human t-PA protein according to claim 1.

**11.** Process for the preparation of DNA having a DNA sequence coding for a human t-PA protein according to claim 1 comprising chemically synthesizing the DNA, or excising a portion of the DNA comprising the codon for the undesired amino acid residue from the parental mature t-PA gene and replacing it with a DNA segment wherein said codon has been substituted with a deoxyribonucleotide triplet coding for the desired amino acid residue, or accomplishing the deoxyribonucleotide substitution by means of site-directed mutagenesis.

**12.** A hybrid vector comprising a promoter and a DNA sequence coding for a human t-PA protein according to claim 1.

**13.** Process for the preparation of a hybrid vector comprising a promoter and a DNA sequence coding for a human t-PA protein according to claim 1 comprising linking the promoter, the human t-PA coding sequence and optionally vector DNA.

**14.** A transformed eukaryotic host cell containing a DNA sequence coding for a human t-PA protein according to claim 1.

**15.** Process for the preparation of transformed eukaryotic host cells comprising transforming eukaryotic host cells with a hybrid vector comprising a promoter and a DNA sequence coding for a human t-PA protein according to claim 1.

**16.** A pharmaceutical composition containing a human t-PA protein according to claim 1 and a pharmaceutically acceptable carrier.

**17.** A human t-PA protein according to claim 1 for use in a process for the therapeutic or prophylactic treatment of the human body.

**18.** The use of a human t-PA protein according to claim 1 for the preparation of a pharmaceutical composition.

**Claims for the following Contracting States : AT, ES, GR**

**1.** Process for the preparation of a human t-PA protein having the amino acid sequence

$$A_{1-116}\text{-Asn-Ser-}Y_1\text{-}A_{120-183}\text{-Asn-Gly-}Y_2\text{-}A_{187-217}\text{-Asn-Pro-}Y_3\text{-}A_{221-447}\text{-Asn-Arg-}Y_4\text{-}A_{451-527} \qquad (I')$$

in which $A_{1-116}$ represents the N-terminal amino acid sequence consisting of amino acids 1 to 116 of mature t-PA, $A_{120-183}$ represents the amino acid sequence consisting of amino acids 120 to 183 of mature t-PA, $A_{187-217}$ represents the amino acid sequence consisting of amino acids 187 to 217 of mature t-PA, $A_{221-447}$ represents the amino acid sequence consisting of amino acids 221 to 447 of mature t-PA, $A_{451-527}$ represents the C-terminal amino acid sequence consisting of amino acids 451 to 527 of mature t-PA, and

a) $Y_1$ and $Y_3$ are Ser, $Y_2$ is a genetically encoded amino acid other than Ser and Thr, and $Y_4$ is Thr; or
b) $Y_1$, $Y_2$ and $Y_3$ are each Ser and $Y_4$ is a genetically encoded amino acid other than Ser and Thr; or
c) $Y_1$, $Y_2$ and $Y_4$ are each independently a genetically encoded amino acid other than Ser and Thr, and $Y_3$ is Ser, in the one-chain or in the two-chain form,
comprising culturing under appropriate nutrient conditions transformed eukaryotic host cells containing a DNA sequence coding for said human t-PA protein, and isolating said human t-PA protein and, if desired, separating a mixture of the one-chain and two-chain forms of the t-PA protein obtained into the individual components, and for the production of the two-chain form which is free of the one-chain form, converting the one-chain form of the t-PA protein produced into the two-chain form.

**2.** Process for the preparation of a t-PA protein according to claim 1 selected from the group consisting of [Ala$^{186}$]-t-PA, [Ala$^{450}$]-t-PA, [Asn$^{119}$, Ala$^{186}$, Asn$^{450}$]-t-PA and [Asn$^{119}$, Ala$^{186}$, Ala$^{450}$]-t-PA.

**3.** Process for the preparation of [Ala$^{186}$]-t-PA according to claim 1.

**4.** Process for the preparation of [Ala$^{450}$]-t-PA according to claim 1.

**5.** Process for the preparation of [Asn$^{119}$, Ala$^{186}$, Ala$^{450}$]-t-PA according to claim 1.

**6.** Process for the preparation of [Asn$^{119}$, Ala$^{186}$, Asn$^{450}$]-t-PA according to claim 1.

**7.** Process for the preparation of a human t-PA protein in the one-chain form, according to claim 1.

**8.** Process for the preparation of a DNA having a DNA sequence coding for a human t-PA protein obtainable according to claim 1 comprising chemically synthesizing the DNA, or excising a portion of the DNA comprising the codon for the undesired amino acid residue from the parental mature t-PA gene and replacing it with a DNA segment wherein said codon has been substituted with a deoxyribonucleotide triplet coding for the desired amino acid residue, or accomplishing the deoxyribonucleotide substitution by means of site-directed mutagenesis.

**9.** Process for the preparation of a hybrid vector comprising a promoter and a DNA sequence coding for a human t-PA protein obtainable according to claim 1 comprising linking the promoter, the human t-PA coding sequence and optionally vector DNA.

**10.** Process for the preparation of transformed eukaryotic host cells comprising transforming eukaryotic host cells with a hybrid vector comprising a promoter and a DNA sequence coding for a human t-PA protein obtainable according to claim 1.

**11.** Process for the preparation of a pharmaceutical composition comprising processing a compound obtainable according to claim 1 with a pharmaceutically acceptable carrier.

**Revendications**
**Revendications pour les Etats contractants suivants : BE, DE, FR, GB, IT, NL, SE, CH, LU**

**1.** Protéines de t-PA humaine ayant la séquence d'acides aminés

$$A_{1-116}\text{-Asn-Ser-}Y_1\text{-}A_{120-183}\text{-Asn-Gly-}Y_2\text{-}A_{187-217}\text{-Asn-Pro-}Y_3\text{-}A_{221-447}\text{-Asn-Arg-}Y_4\text{-}A_{451-527} \qquad \text{(I')}$$

dans laquelle $A_{1-116}$ représente la séquence d'acides aminés N-terminale constituée des acides aminés 1 à 116 du t-PA mûr, $A_{120-183}$ représente la séquence d'acides aminés constituée des acides aminés 120 à 183 du t-PA mûr, $A_{187-217}$ représente la séquence d'acides aminés constituée des acides aminés 187 à 217 du t-PA mûr, $A_{221-447}$ représente la séquence d'acides aminés constituée des acides aminés 221 à 447 du t-PA mûr, $A_{451-527}$ représente la séquence d'acides aminéx C-terminale constituée des acides aminés 451 à 527 du t-PA mûr et
   a) $Y_1$ et $Y_3$ représentent Ser, $Y_2$ est un acide aminé génétiquement encodé autre que Ser et Thr, et $Y_4$ représente Thr ; ou
   b) $Y_1$, $Y_2$ et $Y_3$ représentent chacun Ser et $Y_4$ est un acide aminé génétiquement encodé autre que Ser et Thr ; ou
   c) $Y_1$, $Y_2$ et $Y_4$ représentent chacun indépendamment un acide aminé génétiquement encodé autre que Ser et Thr et $Y_3$ représente Ser, sous forme à une chaîne ou à deux chaînes.

**2.** Protéine de t-PA selon la revendication 1, choisie dans le groupe constitué par

(Ala$^{186}$]-t-PA, [Ala$^{450}$]-t-PA, [Asn$^{119}$, Ala$^{186}$, Asn$^{450}$]-t-PA et [Asn$^{119}$, Ala$^{186}$, Ala$^{450}$]-t-PA.

**3.** [Ala$^{186}$]-t-PA selon la revendication 1.

**4.** [Ala$^{450}$]-t-PA selon la revendication 1.

**5.** [Asn$^{119}$, Ala$^{186}$, Ala$^{450}$]-t-PA selon la revendication 1.

**6.** [Asn$^{119}$, Ala$^{186}$,Asn$^{450}$]-t-PA selon la revendication 1.

**7.** Protéines de t-PA humaines sous forme à une chaîne selon l'une quelconque des revendications 1-6.

**8.** Procédé de préparation d'une protéine de t-PA humaine selon la revendication 1, dans lequel on cultive dans des conditions nutritives appropriées des cellules hôtes eucaryotiques transformées contenant une séquence d'ADN codant pour ladite protéine de t-PA humaine, et on isole ladite protéine de t-PA humaine et, si on le désire, on sépare un mélange des formes à une chaîne et des formes à deux chaînes de la protéine de t-PA obtenue en les composants individuels et pour la production de la forme à deux chaînes qui est dépourvue de la forme à une chaîne, on transforme la forme à une chaîne de la protéine de t-PA produite en la forme à deux chaînes.

**9.** Les protéines de t-PA humaines que l'on peut obtenir par le procédé selon la revendication 8.

**10.** ADN ayant une séquence d'ADN codant pour une protéine de t-PA humaine selon la revendication 1.

**11.** Procédé de préparation d'un ADN ayant une séquence d'ADN codant pour une protéine de t-PA humaine selon la revendication 1 dans lequel on synthétise chimiquement l'ADN, ou on excise une partie de l'ADN comprenant le codon pour le résidu acide aminé indésirable provenant du gène de t-PA mûr parental et on la remplace par un segment d'ADN dans lequel ledit codon a été remplacé par un triplet de désoxyribonucléotide codant pour le résidu acide aminé désiré, ou on accomplit la substitution de désoxyribonucléotide au moyen d'une mutagénèse dirigée.

**12.** Vecteur hybride comprenant un promoteur et une séquence d'ADN codant pour une protéine de t-PA humaine selon la revendication 1.

**13.** Procédé de préparation d'un vecteur hybride comprenant un promoteur et une séquence d'ADN codant pour une protéine de t-PA humaine selon la revendication 1, dans lequel on ligature le promoteur, la séquence de codage de t-PA humaine, et si on le désire l'ADN de vecteur.

**14.** Cellule hôte eucaryotique transformée comprenant une séquence d'ADN codant pour une protéine de t-PA humaine selon la revendication 1.

**15.** Procédé de préparation de cellules hôtes eucaryotiques transformées dans lequel on transforme les cellules hôtes eucaryotiques avec un vecteur hybride comprenant un promoteur et une séquence d'ADN codant pour une protéine de t-PA humaine selon la revendication 1.

**16.** Composition pharmaceutique comprenant une protéine de t-PA humaine selon la revendication 1 et un support pharmaceutiquement acceptable.

**17.** Protéine de t-PA humaine selon la revendication 1 pour utilisation dans un procédé de traitement thérapeutique ou prophylactique du corps humain.

**18.** Application d'une protéine de t-PA humaine selon la revendication 1 à la préparation d'une composition pharmaceutique.

**Revendications pour les Etats contractants suivants : AT, ES, GR**

**1.** Procédé de préparation d'une protéine de t-PA humaine ayant la séquence d'acides aminés

$$A_{1-116}\text{-Asn-Ser-}Y_1\text{-}A_{120-183}\text{-Asn-Gly-}Y_2\text{-}A_{187-217}\text{-Asn-Pro-}Y_3\text{-}A_{221-447}\text{-Asn-Arg-}Y_4\text{-}A_{451-527} \qquad (I')$$

dans laquelle $A_{1-116}$ représente la séquence d'acides aminés N-terminale constituée des acides aminés 1 à 116 du t-PA mûr, $A_{120-183}$ représente la séquence d'acides aminés constituée des acides aminés 120 à 183 du t-PA mûr, $A_{187-217}$ représente la séquence d'acides aminés constituée des acides aminés 187 à 217 du t-PA mûr, $A_{221-447}$ représente la séquence d'acides aminés constituée des acides aminés 221 à 447 du t-PA mûr, $A_{451-527}$ représente la séquence d'acides aminéx C-terminale constituée des acides aminés 451 à 527 du t-PA mûr et

a) $Y_1$ et $Y_3$ représentent Ser, $Y_2$ est un acide aminé génétiquement encodé autre que Ser et Thr, et $Y_4$ représente Thr ; ou

b) $Y_1$, $Y_2$ et $Y_3$ représentent chacun Ser et $Y_4$ est un acide aminé génétiquement encodé autre que Ser et Thr ; ou

c) $Y_1$, $Y_2$ et $Y_4$ représentent chacun indépendamment un acide aminé génétiquement encodé autre que Ser et Thr et $Y_3$ représente Ser, sous forme à une chaîne ou à deux chaînes, dans lequel on cultive dans des conditions nutritives appropriées des cellules hôtes eucaryotiques transformées contenant une séquence d'ADN codant pour ladite protéine de t-PA humaine, et on isole ladite protéine de t-PA humaine et, si on le désire, on sépare un mélange des formes à une chaîne et des formes à deux chaînes de la protéine de t-PA obtenue en les composants individuels et pour la production de la forme à deux chaînes qui est dépourvue de la forme à une chaîne, on transforme la forme à une chaîne de la protéine de t-PA produite en la forme à deux chaînes.

2. Procédé de préparation d'une protéine de t-PA selon la revendication 1 choisie dans le groupe constitué par
[Ala$^{186}$]-t-PA, [Ala$^{450}$]-t-PA,
[Asn$^{119}$, Ala$^{186}$, Asn$^{450}$]-t-PA et [Asn$^{119}$, Ala$^{186}$, Ala$^{450}$]-t-PA.

3. Procédé de préparation de

[Ala$^{186}$]-t-PA

selon la revendication 1.

4. Procédé de préparation de

[Ala$^{450}$]-t-PA

selon la revendication 1.

5. Procédé de préparation de

[Asn$^{119}$, Ala$^{186}$, Ala$^{450}$]-t-PA

selon la revendication 1.

6. Procédé de préparation de

[Asn$^{119}$, Ala$^{186}$, Asn$^{450}$]-t-PA

selon la revendication 1.

7. Procédé de préparation d'une protéine de t-PA humaine sous la forme à une chaîne selon la revendication 1.

8. Procédé de préparation d'un ADN ayant une séquence d'ADN codant pour une protéine de t-PA humaine que l'on peut obtenir selon la revendication 1, dans lequel on synthétise chimiquement l'ADN, ou on excise une partie de l'ADN comprenant le codon pour le résidu acide aminé indésirable provenant du gène de t-PA mûr parental, et on la remplace par un segment d'ADN dans lequel ledit codon a été remplacé par un triplet de désoxyribonucléotide codant pour le résidu acide aminé désiré, ou on accomplit la substitution de désoxyribonucléotide au moyen d'une mutagénèse dirigée.

9. Procédé de préparation d'un vecteur hybride comprenant un promoteur et une séquence d'ADN codant pour une protéine de t-PA humaine que l'on peut obtenir selon la revendication 1, dans lequel on ligature le promoteur, la séquence de codage de t-PA humaine et, si on le désire, l'ADN vecteur.

10. Procédé de préparation de cellules hôtes eucaryotiques transformées dans lequel on transforme des cellules hôtes eucaryotiques avec un vecteur hybride comprenant un promoteur et une séquence d'ADN codant pour une protéine de t-PA humaine que l'on peut obtenir selon la revendication 1.

11. Procédé de préparation d'une composition pharmaceutique dans lequel on traite un composé que l'on peut obtenir selon la revendication 1 avec un support pharmaceutiquement acceptable.

**Patentansprüche**
**Patentansprüche für folgende Vertragsstaaten : BE, DE, FR, GB, IT, NL, SE, CH, LU**

1. Human-t-PA-Proteine mit der Aminosäuresequenz

$A_{1-116}$-Asn-Ser-$Y_1$-$A_{120-183}$-Asn-Gly-$Y_2$-$A_{187-217}$-Asn-Pro-$Y_3$-$A_{221-447}$-Asn-Arg-$Y_4$-$A_{451-527}$ (I')
in der $A_{1-116}$ die N-terminale Aminosäuresequenz repräsentiert, die aus den Aminosäuren 1 bis 116 von reifem t-PA besteht, $A_{120-183}$ die Aminosäuresequenz repräsentiert, die aus den Aminosäuren 120 bis 183 von reifem t-PA besteht. $A_{187-217}$ die Aminosäuresequenz darstellt, die aus den Aminosäuren 187 bis 217 von reifem t-PA besteht, $A_{221-447}$ die Aminosäuresequenz darstellt, die aus den Aminosäuren 221 bis 447 von reifem t-PA besteht, $A_{451-527}$ die C-terminale Aminosäuresequenz darstellt, die aus den Aminosäuren 451 bis 527 von reifem t-PA besteht, und a) $Y_1$ und $Y_3$ Ser sind, $Y_2$ eine andere genetisch codierte Aminosäure als Ser und Thr ist, und $Y_4$ Thr ist; oder b) $Y_1$, $Y_2$ und $Y_3$ jedes Ser sind und $Y_4$ eine andere genetisch codierte Aminosäure als Ser und Thr ist; oder c) $Y_1$, $Y_2$ und $Y_4$ jedes unabhängig eine andere genetisch codierte Aminosäure als Ser und Thr sind und $Y_3$ Ser ist, in der Einketten- oder in der Zweiketten-Form.

2. t-PA-Protein nach Anspruch 1, ausgewählt aus der Gruppe bestehend aus [$Ala^{186}$]-t-PA, [$Ala^{450}$]-t-PA, [$Asn^{119}$,$Ala^{186}$,$Asn^{450}$]-t-PA und [$Asn^{119}$,$Ala^{186}$,$Ala^{450}$]-t-PA.

3. [$Ala^{186}$]-t-PA nach Anspruch 1.

4. [$Ala^{450}$]-t-PA nach Anspruch 1.

5. [$Asn^{119}$,$Ala^{186}$,$Ala^{450}$]-t-PA nach Anspruch 1.

6. [$Asn^{119}$,$Ala^{186}$,$Asn^{450}$]-t-PA nach Anspruch 1.

7. Human-t-PA-Proteine in der Einketten-Form nach irgendeinem der Ansprüche 1 - 6.

8. Verfahren zur Herstellung eines Human-t-PA-Proteins nach Anspruch 1, umfassend das Kultivieren transformierter eukaryotischer Wirtszellen, die eine für das Human-t-PA-Protein codierende DNA-Sequenz enthalten, unter geeigneten Nährbedingungen und Isolieren des Human-t-PA-Proteins und, falls gewünscht, Trennen einer erhaltenen Mischung der Einketten- und Zweiketten-Formen des t-PA-Proteins in die individuellen Komponenten und für die Herstellung der Zweiketten-Form, die frei von Einketten-Form ist, Umwandeln der Einketten-Form des hergestellten-t-PA-Proteins in die Zweiketten-Form.

9. Human-t-PA-Proteine, die nach dem Verfahren nach Anspruch 8 erhältlich sind.

10. DNA mit einer für ein Human-t-PA-Protein nach Anspruch 1 codierenden DNA-Sequenz.

11. Verfahren zur Herstellung von DNA mit einer für ein Human-t-PA-Protein nach Anspruch 1 codierenden Sequenz, umfassend chemische Synthese der DNA oder Exzisieren eines Teils der DNA umfassend das Codon für den unerwünschten Aminosäurerest vom Stammgen von reifem t-PA und Ersetzen durch ein DNA-Segment, in dem besagtes Codon durch ein Desoxyribonucleotid-Triplett, das für den gewünschten Aminosäure-Rest codiert, substituiert wurde, oder Durchführung der Desoxyribonucleotid-Substitution durch ortsgerichtete Mutagenese.

12. Hybridvektor, umfassend einen Promotor und eine DNA-Sequenz, die für ein Human-t-PA-Protein nach Anspruch 1 codiert.

13. Verfahren zur Herstellung eines Hybridvektors, umfassend einen Promotor und eine DNA-Sequenz, die für ein Human-t-PA-Protein nach Anspruch 1 codiert, umfassend das Verknüpfen des Promotors, der Human-t-PA codierenden Sequenz und wahlweise Vektor-DNA.

14. Transformierte eukaryotische Wirtszelle, enthaltend eine DNA-Sequenz, die für ein Human-t-PA-Protein nach Anspruch 1 codiert.

15. Verfahren zur Herstellung transformierter eukaryotischer Wirtszellen, umfassend das Transformieren eukaryotischer Wirtszellen mit einem Hybridvektor, umfassend einen Promotor und eine DNA-Sequenz, die für ein Human-t-PA-Protein nach Anspruch 1 codiert.

16. Pharmazeutische Zusammensetzung, die ein Human-t-PA-Protein nach Anspruch 1 und einen pharmazeutisch annehmbaren Träger enthält.

17. Human-t-PA-Protein nach Anspruch 1 zur Verwendung in einem Verfahren zur therapeutischen oder prophylaktischen Behandlung des menschlichen Körpers.

18. Verwendung eines Human-t-PA-Proteins nach Anspruch 1 zur Herstellung einer pharmazeutischen Zusammensetzung.

**Patentansprüche für folgende Vertragsstaaten : AT, ES, GR**

1. Verfahren zur Herstellung eines Human-t-PA-Proteins mit der Aminosäuresequenz
$A_{1-116}$-Asn-Ser-$Y_1$-$A_{120-183}$–Asn-Gly-$Y_2$-$A_{187-217}$-Asn-Pro-$Y_3$-$A_{221-447}$-Asn-Arg-$Y_4$-$A_{451-527}$ (I')
in der $A_{1-116}$ die N-terminale Aminosäuresequenz repräsentiert, die aus den Aminosäuren 1 bis 116 von reifem t-PA besteht, $A_{120-183}$ die Aminosäuresequenz repräsentiert, die aus den Aminosäuren 120 bis 183 von reifem t-PA besteht, $A_{187-217}$ die Aminosäuresequenz darstellt, die aus den Aminosäuren 187 bis 217 von reifem t-PA besteht, $A_{221-447}$ die Aminosäuresequenz darstellt, die aus den Aminosäuren 221 bis 447 von reifem t-PA besteht, $A_{451-527}$ die C-terminale Aminosäuresequenz darstellt, die aus den Aminosäuren 451 bis 527 von reifem t-PA besteht, und a) $Y_1$ und $Y_3$ Ser sind, $Y_2$ eine andere genetisch codierte Aminosäure als Ser und Thr ist, und $Y_4$ Thr ist; oder b) $Y_1$, $Y_2$ und $Y_3$ jedes Ser sind und $Y_4$ eine andere genetisch codierte Aminosäure als Ser und Thr ist; oder c) $Y_1$, $Y_2$ und $Y_4$ jedes unabhängig eine andere genetisch codierte Aminosäure als Ser und Thr sind und $Y_3$ Ser ist, in der Einketten- oder in der Zweiketten-Form, umfassend das Kultivieren transformierter eukaryotischer Wirtszellen, die eine für das Human-t-PA-Protein codierende DNA-Sequenz enthalten, untergeeigneten Nährbedingungen und Isolieren des Human-t-PA-Proteins und, falls gewünscht, Trennen einer erhaltenen Mischung der Einketten- und Zweiketten-Formen des t-PA-Proteins in die individuellen Komponenten und für die Herstellung der Zweiketten-Form, die frei von Einketten-Form ist, Umwandeln der Einketten-Form des hergestellten t-PA-Proteins in die Zweiketten-Form.

2. Verfahren zur Herstellung eines t-PA-Proteins nach Anspruch 1, ausgewählt aus der Gruppe bestehend aus [$A^{186}$]-t-PA, [$Ala^{450}$]-t-PA, [$Asn^{119}$, $Ala^{186}$, $Asn^{450}$]-t-PA und [$Asn^{119}$, $Ala^{186}$, $Ala^{450}$]-t-PA.

3. Verfahren zur Herstellung von [$Ala^{186}$]-t-PA nach Anspruch 1.

4. Verfahren zur Herstellung von [$Ala^{450}$]-t-PA nach Anspruch 1.

5. Verfahren zur Herstellung von [$Asn^{119}$,$Ala^{186}$,$Ala^{450}$]-t-PA nach Anspruch 1.

6. Verfahren zur Herstellung von [$Asn^{119}$,$Ala^{186}$,$Asn^{450}$]-t-PA nach Anspruch 1.

7. Verfahren zur Herstellung eines Human-t-PA-Proteins in der Einketten-Form nach Anspruch 1.

8. Verfahren zur Herstellung einer DNA mit einer für ein Human-t-PA-Protein, das nach Anspruch 1 erhältlich ist, codierenden Sequenz, umfassend chemische Synthese der DNA oder Exzisieren eines Teils der DNA umfassend das Codon für den unerwünschten Aminosäurerest vom Stammgen von reifem t-PA und Ersetzen durch ein DNA-Segment, in dem besagtes Codon durch ein Desoxyribonucleotid-Triplett, das für den gewünschten Aminosäure-Rest codiert, substituiert wurde, oder Durchführung der Desoxyribonucleotid-Substitution durch ortsgerichtete Mutagenese.

**9.** Verfahren zur Herstellung eines Hybridvektors, enthaltend einen Promotor und eine DNA-Sequenz, die für ein nach Anspruch 1 erhältliches Human-t-PA-Protein codiert, umfassend das Verknüpfen des Promotors, der Human-t-PA codierenden Sequenz und wahlweise Vektor-DNA.

**10.** Verfahren zur Herstellung transformierter eukaryotischer Wirtszellen, umfassend das Transformieren eukaryotischer Wirtszellen mit einem Hybridvektor, umfassend einen Promotor und eine DNA-Sequenz, die für ein Human-t-PA-Protein, das nach Anspruch 1 erhältlich ist, codiert.

**11.** Verfahren zur Herstellung einer pharmazeutischen Zusammensetzung, umfassend das Verarbeiten einer nach Anspruch 1 erhältlichen Verbindung mit einem pharmazeutisch annehmbaren Träger.

# *Fig. 1*  Nucleotide sequence and deduced amino acid sequence of mature t-PA.

```
                                                                              17
      SER TYR GLN VAL ILE CYS ARG ASP GLU LYS THR GLN MET ILE TYR GLN GLN
    1 TCT TAC CAA GTG ATC TGC AGA GAT GAA AAA ACG CAG ATG ATA TAC CAG CAA

                                                                              34
      HIS GLN SER TRP LEU ARG PRO VAL LEU ARG SER ASN ARG VAL GLU TYR CYS
   52 CAT CAG TCA TGG CTG CGC CCT GTG CTC AGA AGC AAC CGG GTG GAA TAT TGC

                                                                              51
      TRP CYS ASN SER GLY ARG ALA GLN CYS HIS SER VAL PRO VAL LYS SER CYS
  103 TGG TGC AAC AGT GGC AGG GCA CAG TGC CAC TCA GTG CCT GTC AAA AGT TGC

                                                                              68
      SER GLU PRO ARG CYS PHE ASN GLY GLY THR CYS GLN GLN ALA LEU TYR PHE
  154 AGC GAG CCA AGG TGT TTC AAC GGG GGC ACC TGC CAG CAG GCC CTG TAC TTC

                                                                              85
      SER ASP PHE VAL CYS GLN CYS PRO GLU GLY PHE ALA GLY LYS CYS CYS GLU
  205 TCA GAT TTC GTG TGC CAG TGC CCC GAA GGA TTT GCT GGG AAG TGC TGT GAA

                                                                             102
      ILE ASP THR ARG ALA THR CYS TYR GLU ASP GLN GLY ILE SER TYR ARG GLY
  256 ATA GAT ACC AGG GCC ACG TGC TAC GAG GAC CAG GGC ATC AGC TAC AGG GGC

                                                                             119
      THR TRP SER THR ALA GLU SER GLY ALA GLU CYS THR ASN TRP ASN SER SER
  307 ACG TGG AGC ACA GCG GAG AGT GGC GCC GAG TGC ACC AAC TGG AAC AGC AGC

                                                                             136
      ALA LEU ALA GLN LYS PRO TYR SER GLY ARG ARG PRO ASP ALA ILE ARG LEU
  358 GCG TTG GCC CAG AAG CCC TAC AGC GGG CGG AGG CCA GAC GCC ATC AGG CTG

                                                                             153
      GLY LEU GLY ASN HIS ASN TYR CYS ARG ASN PRO ASP ARG ASP SER LYS PRO
  409 GGC CTG GGG AAC CAC AAC TAC TGC AGA AAC CCA GAT CGA GAC TCA AAG CCC

                                                                             170
      TRP CYS TYR VAL PHE LYS ALA GLY LYS TYR SER SER GLU PHE CYS SER THR
  460 TGG TGC TAC GTC TTT AAG GCG GGG AAG TAC AGC TCA GAG TTC TGC AGC ACC

                                                                             187
      PRO ALA CYS SER GLU GLY ASN SER ASP CYS TYR PHE GLY ASN GLY SER ALA
  511 CCT GCC TGC TCT GAG GGA AAC AGT GAC TGC TAC TTT GGG AAT GGG TCA GCC

                                                                             204
      TYR ARG GLY THR HIS SER LEU THR GLU SER GLY ALA SER CYS LEU PRO TRP
  562 TAC CGT GGC ACG CAC AGC CTC ACC GAG TCG GGT GCC TCC TGC CTC CCG TGG

                                                                             221
      ASN SER MET ILE LEU ILE GLY LYS VAL TYR THR ALA GLN ASN PRO SER ALA
  613 AAT TCC ATG ATC CTG ATA GGC AAG GTT TAC ACA GCA CAG AAC CCC AGT GCC

                                                                             238
      GLN ALA LEU GLY LEU GLY LYS HIS ASN TYR CYS ARG ASN PRO ASP GLY ASP
  664 CAG GCA CTG GGC CTG GGC AAA CAT AAT TAC TGC CGG AAT CCT GAT GGG GAT

                                                                             255
      ALA LYS PRO TRP CYS HIS VAL LEU LYS ASN ARG ARG LEU THR TRP GLU TYR
  715 GCC AAG CCC TGG TGC CAC GTG CTG AAG AAC CGC AGG CTG ACG TGG GAG TAC

                                                                             272
      CYS ASP VAL PRO SER CYS SER THR CYS GLY LEU ARG GLN TYR SER GLN PRO
  766 TGT GAT GTG CCC TCC TGC TCC ACC TGC GGC CTG AGA CAG TAC AGC CAG CCT
```

35

## Fig. 1 (Continued)

```
        GLN PHE ARG↓ILE LYS GLY GLY LEU PHE ALA ASP ILE ALA SER HIS PRO TRP 289
        CAG TTT CGC ATC AAA GGA GGG CTC TTC GCC GAC ATC GCC TCC CAC CCC TGG

        GLN ALA ALA ILE PHE ALA LYS HIS ARG ARG SER PRO GLY GLU ARG PHE LEU 306
        CAG GCT GCC ATC TTT GCC AAG CAC AGG AGG TCG CCC GGA GAG CGG TTC CTG

        CYS GLY GLY ILE LEU ILE SER SER CYS TRP ILE LEU SER ALA ALA HIS CYS 323
        TGC GGG GGC ATA CTC ATC AGC TCC TGC TGG ATT CTC TCT GCC GCC CAC TGC

        PHE GLN GLU ARG PHE PRO PRO HIS HIS LEU THR VAL ILE LEU GLY ARG THR 340
        TTC CAG GAG AGG TTT CCG CCC CAC CAC CTG ACG GTG ATC TTG GGC AGA ACA

        TYR ARG VAL VAL PRO GLY GLU GLU GLU GLN LYS PHE GLU VAL GLU LYS TYR 357
        TAC CGG GTG GTC CCT GGC GAG GAG GAG CAG AAA TTT GAA GTC GAA AAA TAC

        ILE VAL HIS LYS GLU PHE ASP ASP ASP THR TYR ASP ASN ASP ILE ALA LEU 374
        ATT GTC CAT AAG GAA TTC GAT GAT GAC ACT TAC GAC AAT GAC ATT GCG CTG

        LEU GLN LEU LYS SER ASP SER SER ARG CYS ALA GLN GLU SER SER VAL VAL 391
        CTG CAG CTG AAA TCG GAT TCG TCC CGC TGT GCC CAG GAG AGC AGC GTG GTC

        ARG THR VAL CYS LEU PRO PRO ALA ASP LEU GLN LEU PRO ASP TRP THR GLU 408
        CGC ACT GTG TGC CTT CCC CCG GCG GAC CTG CAG CTG CCG GAC TGG ACG GAG

        CYS GLU LEU SER GLY TYR GLY LYS HIS GLU ALA LEU SER PRO PHE TYR SER 425
        TGT GAG CTC TCC GGC TAC GGC AAG CAT GAG GCC TTG TCT CCT TTC TAT TCG

        GLU ARG LEU LYS GLU ALA HIS VAL ARG LEU TYR PRO SER SER ARG CYS THR 442
        GAG CGG CTG AAG GAG GCT CAT GTC AGA CTG TAC CCA TCC AGC CGC TGC ACA

        SER GLN HIS LEU LEU ASN ARG THR VAL THR ASP ASN MET LEU CYS ALA GLY 459
        TCA CAA CAT TTA CTT AAC AGA ACA GTC ACC GAC AAC ATG CTG TGT GCT GGA

        ASP THR ARG SER GLY GLY PRO GLN ALA ASN LEU HIS ASP ALA CYS GLN GLY 476
        GAC ACT CGG AGC GGC GGG CCC CAG GCA AAC TTG CAC GAC GCC TGC CAG GGC

        ASP SER GLY GLY PRO LEU VAL CYS LEU ASN ASP GLY ARG MET THR LEU VAL 493
        GAT TCG GGA GGC CCC CTG GTG TGT CTG AAC GAT GGC CGC ATG ACT TTG GTG

        GLY ILE ILE SER TRP GLY LEU GLY CYS GLY GLN LYS ASP VAL PRO GLY VAL 510
        GGC ATC ATC AGC TGG GGC CTG GGC TGT GGA CAG AAG GAT GTC CCG GGT GTG

        TYR THR LYS VAL THR ASN TYR LEU ASP TRP ILE ARG ASP ASN MET ARG PRO 527
        TAC ACA AAG GTT ACC AAC TAC CTA GAC TGG ATT CGT GAC AAC ATG CGA CCG
```

# _Fig. 2_  DNA sequences at the sites of mutation and corresponding t-PA protein sequences

Mature t-PA:

| 1 | | | 119 | | 186 | | 220 | | 450 | 527 |
|---|---|---|---|---|---|---|---|---|---|---|
| Ser————————Asn | Ser | Ser————————Asn | Gly | Ser———Asn | Pro | Ser————Asn | Arg | Thr—————————Pro |
| TCT————————AAC | AGC | AGC————————AAT | GGG | TCA———AAC | CCC | AGT————AAC | AGA | ACA—————————CCG |

1. [Asn$^{119}$]-t-PA

| 1 | | | 119 | | 186 | | 220 | | 450 | 527 |
| Ser————————Asn | Ser | Asn————————Asn | Gly | Ser———Asn | Pro | Ser————Asn | Arg | Thr—————————Pro |
| TCT————————AAC | AGC | AAC————————AAT | GGG | TCA———AAC | CCC | AGT————AAC | AGA | ACA—————————CCG |

2. [Ala$^{186}$]-t-PA

| 1 | | | 119 | | 186 | | 220 | | 450 | 527 |
| Ser————————Asn | Ser | Ser————————Asn | Gly | Ala———Asn | Pro | Ser————Asn | Arg | Thr—————————Pro |
| TCT————————AAC | AGC | AGC————————AAT | GGG | GCA———AAC | CCC | AGT————AAC | AGA | ACA—————————CCG |

3. [Ala$^{450}$]-t-PA

| 1 | | | 119 | | 186 | | 220 | | 450 | 527 |
| Ser————————Asn | Ser | Ser————————Asn | Gly | Ser———Asn | Pro | Ser————Asn | Arg | Ala—————————Pro |
| TCT————————AAC | AGC | AGC————————AAT | GGG | TCA———AAC | CCC | AGT————AAC | AGA | GCA—————————CCG |

**Fig. 2** (Continued)

4. [Asn$^{119}$, Ala$^{186}$]-t-PA

| 1 | | | 119 | | 186 | | 220 | | 450 | 527 |

Ser————————Asn Ser Asn————————Asn Gly Ala————Asn Pro Ser————Asn Arg Thr————————Pro

TCT————————AAC AGC AAC————————AAT GGG GCA————AAC CCC AGT————AAC AGA ACA————————CCG


5. [Asn$^{119}$, Ala$^{186}$, Asn$^{450}$]-t-PA

| 1 | | | 119 | | 186 | | 220 | | 450 | 527 |

Ser————————Asn Ser Asn————————Asn Gly Ala————Asn Pro Ser————Asn Arg Asn————————Pro

TCT————————AAC AGC AAC————————AAT GGG GCA————AAC CCC AGT————AAC AGA AAC————————CCG


6. [Asn$^{119}$, Ala$^{186}$, Ala$^{450}$]-t-PA

| 1 | | | 119 | | 186 | | 220 | | 450 | 527 |

Ser————————Asn Ser Asn————————Asn Gly Ala————Asn Pro Ser————Asn Arg Ala————————Pro

TCT————————AAC AGC AAC————————AAT GGG GCA————AAC CCC AGT————AAC AGA GCA————————CCG


7. [Asn$^{119}$, Ala$^{186}$, Asn$^{220}$, Ala$^{450}$]-t-PA

| 1 | | | 119 | | 186 | | 220 | | 450 | 527 |

Ser————————Asn Ser Asn————————Asn Gly Ala————Asn Pro Asn————Asn Arg Ala————————Pro

TCT————————AAC AGC AAC————————AAT GGG GCA————AAC CCC AAT————AAC AGA GCA————————CCG

Fig. 3    Activity of mutant t - PAs in the clot lysis assay.

*Fig. 4* Immunodetection of mutant t-PAs
(Western blots)

1: authentic human melanoma t-PA

2: [Asn$^{119}$, Ala$^{186}$, Asn$^{450}$]-t-PA

3: [Asn$^{119}$, Ala$^{186}$]-t-PA

4: [Asn$^{119}$]-t-PA

5: yeast wild-type t-PA